(19) Europäisches Patentamt
European Patent Office
Office européen des brevets

(11) **EP 4 382 125 A1**

(12) **EUROPEAN PATENT APPLICATION**
published in accordance with Art. 153(4) EPC

(43) Date of publication:
**12.06.2024 Bulletin 2024/24**

(21) Application number: **22853046.5**

(22) Date of filing: **02.08.2022**

(51) International Patent Classification (IPC):
**A61K 45/00** (2006.01)　　**A61K 31/155** (2006.01)
**A61K 47/26** (2006.01)　　**A61K 47/32** (2006.01)
**A61L 2/18** (2006.01)　　**A61P 17/00** (2006.01)

(52) Cooperative Patent Classification (CPC):
**A61K 31/155; A61K 45/00; A61K 47/26;**
**A61K 47/32; A61L 2/18; A61P 17/00**

(86) International application number:
**PCT/JP2022/029618**

(87) International publication number:
**WO 2023/013627 (09.02.2023 Gazette 2023/06)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **06.08.2021　JP 2021129996**

(71) Applicant: **Otsuka Pharmaceutical Factory, Inc.**
**Naruto-shi, Tokushima 772-8601 (JP)**

(72) Inventors:
• **SHIOZAKI, Mari**
**Naruto-shi, Tokushima 772-8601 (JP)**
• **HAGI, Akifumi**
**Naruto-shi, Tokushima 772-8601 (JP)**
• **NISHIOKA, Hisae**
**Naruto-shi, Tokushima 772-8601 (JP)**

(74) Representative: **Hamer, Christopher K.**
**Mathys & Squire**
**The Shard**
**32 London Bridge Street**
**London SE1 9SG (GB)**

(54) **DISINFECTING COMPOSITION**

(57)　The object of the present invention is to provide an antiseptic composition that can reduce the risk of an incise drape coming off from the skin, without adversely affecting microbicidal activity, even when the incise drape is affixed to skin surface after application of the antiseptic composition, and/or exerts more effective microbicidal activity. A composition comprising a microbicide and a sugar alcohol is used as the antiseptic composition.

[Fig. 3]

EP 4 382 125 A1

## Description

### Technical Field

The present invention relates to an antiseptic composition (disinfecting composition) comprising a microbicide and a sugar alcohol (hereinafter, also referred to as the "present antiseptic composition").

### Background Art

Bacterial infection at an operation site in performing surgery (surgical site infection; (SSI)) is often caused by bacteria attached or residing on the skin. Before operation, the skin is therefore disinfected. However, it is difficult to completely remove bacteria, though the number of bacteria present on the skin can be decreased by skin antisepsis. As an operation time passes, skin resident microbiota reproliferates and might also contaminate an operative field.

Accordingly, the Japanese Association for Operative Medicine has released "Practical Guidelines for Operative Medicine (revised)" (see, for example, non-patent document 1). This guideline states that incise drapes reduce the influence of skin resident microbiota which is responsible for SSI in the clean operation of cardiac surgery, cranial nerve surgery, orthopedic surgery, or the like.

Meanwhile, an antiseptic composition supplemented with a colorant is used so as to render an application site recognizable in disinfecting the skin before operation. However, such a colorant added into the antiseptic composition reacts with a microbicide serving as an active ingredient to form an insoluble salt of the microbicide, which disadvantageously reduces an antiseptic effect. In order to solve this problem, it is known that the formation of an insoluble salt of the microbicide is suppressed by adding a solubilizing agent to the antiseptic composition. However, a solubilizing agent reduces the adhesive force of incise drape to the skin after antiseptic application.

It has been reported that a water-soluble hot-melt adhesive comprising polyvinyl alcohol having a specific average degree of polymerization and a specific degree of saponification, and water is supplemented with mannitol as a plasticizer of polyvinyl alcohol (patent document 1). Also, a wet adhesive composition has been reported which comprises a water-soluble polymer such as polyvinyl alcohol or polyvinylpyrrolidone, a sugar alcohol, such as mannitol, having compatibility with the water-soluble polymer, and zeolite (patent document 2). However, it has not been known so far that: mannitol added to an antiseptic composition enhances the adhesion force between an incise drape and skin surface to which the antiseptic composition has been applied; and mannitol and polyvinyl alcohol or polyvinylpyrrolidone added to an antiseptic composition further increase such an enhancing effect or increase microbicidal activity.

Prior Art Documents

Patent Documents

[Patent Document 1] Japanese unexamined Patent Application Publication No. 6-49423
[Patent Document 2] Japanese unexamined Patent Application Publication No. 2000-159633

### Non-patent Documents

[Non-patent Document 1] "Practical Guidelines for Operative Medicine", Journal of Japanese Association for Operative Medicine, Vol. 35, Suppl., 2013

### Summary of the Invention

### Object to be Solved by the Invention

An object of the present invention is to provide an antiseptic composition that can reduce the risk of an incise drape coming off from the skin, without adversely affecting microbicidal activity, even when the incise drape is affixed to skin surface after application of the antiseptic composition, and/or exerts more effective microbicidal activity.

### Means to Solve the Object

The present inventors are continuing diligent studies to attain the object. During the process, the present inventors have found that the addition of a sugar alcohol to an antiseptic composition comprising a microbicide enhances the adhesion force of an incise drape on skin surface to which the antiseptic composition has been applied, as compared with the case of adding no sugar alcohol or the case of adding an adhesive agent. The present inventors have also confirmed that the addition of both a sugar alcohol and an adhesive agent to an antiseptic composition comprising a microbicide further enhances the adhesion force of an incise drape on skin surface to which the antiseptic composition has been applied, and tends to increase microbicidal activity on viable microbes such as *Candida albicans,* as compared with the case of adding the sugar alcohol or the adhesive agent alone. Further, they have confirmed that the adhesion force enhancing effect of the incise drape by the present antiseptic composition is exerted without relying on the types of incise drape, and that the adhesion force by the present antiseptic composition is also exerted when using other medical tapes, not limiting to incise drape. The present invention has been completed on the basis of these findings.

Specifically, the present invention is as follows.

[1] An antiseptic composition comprising a microbicide and a sugar alcohol.
[2] The antiseptic composition according to the above [1], wherein the sugar alcohol has an action of enhancing an adhesion force of an incise drape on skin surface after application of the antiseptic composition.
[3] The antiseptic composition according to the above [1] or [2], wherein the sugar alcohol is D-mannitol or erythritol.
[4] The antiseptic composition according to any one of the above [1] to [3], further comprising an adhesive agent.
[5] The antiseptic composition according to the above [4], wherein the adhesive agent has an action of enhancing an adhesion force of an incise drape on skin surface after application of the antiseptic composition.
[6] The antiseptic composition according to the above [4] or [5], wherein a concentration of the adhesive agent is 2% (w/v) or less.
[7] The antiseptic composition according to any one of the above [4] to [6], wherein the adhesive agent is polyvinyl alcohol or polyvinylpyrrolidone.
[8] The antiseptic composition according to any one of the above [1] to [7], wherein the microbicide is olanexidine gluconate.

Examples of other embodiments of the present invention can include:

a method for disinfecting a subject, comprising the step of applying a liquid type of the present antiseptic composition to the subject, wherein the method optionally comprises the step of dissolving a non-liquid type of the present antiseptic composition in a solvent to prepare the liquid type of the present antiseptic composition;
a combination of a microbicide and a sugar alcohol for use in the antisepsis of a subject;
use of a microbicide and a sugar alcohol in the preparation of the present antiseptic composition; and
a method for preparing the present antiseptic composition, comprising the step of preparing the present antiseptic composition by adding a sugar alcohol to a microbicide or adding a microbicide to a sugar alcohol.

Effect of the Invention

The addition of a sugar alcohol to an antiseptic composition comprising a microbicide can enhance the adhesion force of an incise drape on skin surface to which the antiseptic composition has been applied, as compared with the case of adding no sugar alcohol regardless of the types of incise drape. Further, the addition of a sugar alcohol to an antiseptic composition comprising a microbicide can enhance equally or more the adhesion force of an incise drape on skin surface to which the antiseptic composition is applied, regardless of the types of incise drape, as compared with the case of adding an adhesive agent. Hence, clean operation with a reduced risk of SSI is achieved because of low possibility that an incise drape comes off from the skin, even if the incise drape is affixed after application of the present antiseptic composition (particularly, the present antiseptic composition comprising a solubilizing agent) and incision operation is conducted thereon.

The addition of both a sugar alcohol and an adhesive agent to an antiseptic composition comprising a microbicide enhances the adhesion force of an incise drape on skin surface to which the antiseptic composition has been applied, and tends to increase microbicidal activity, as compared with the case of adding the sugar alcohol or the adhesive agent alone. Hence, the present antiseptic composition further comprising the adhesive agent can keep lower the possibility that an incise drape comes off from the skin, even if the incise drape is affixed after application of the present antiseptic composition and incision operation is conducted thereon. In addition, this present antiseptic composition can be expected to have a higher preventive effect on infectious disease ascribable to viable microbes such as *Candida albicans.*

Further, by using the present antiseptic composition, the adhesion force of other medical tapes other than incise drape (for example, surgical tape) can be also enhanced.

**Brief Description of Drawings**

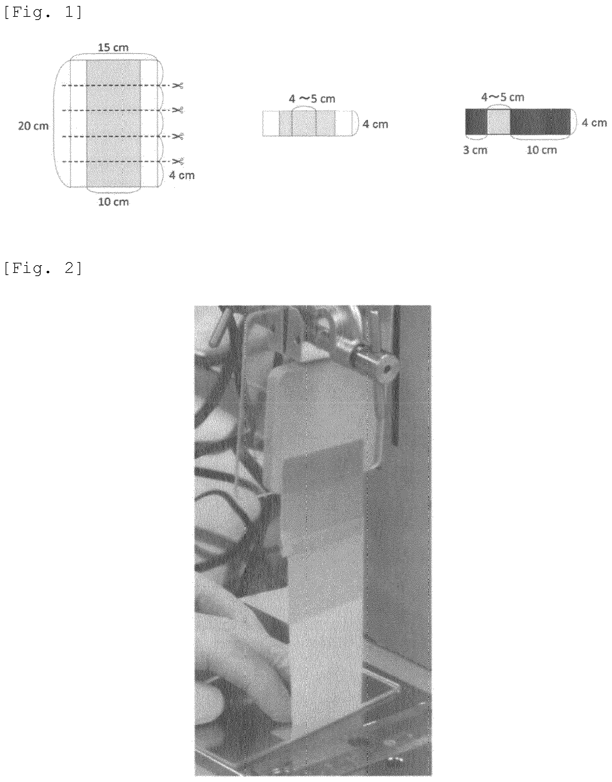

[Figure 1] Figure 1 is a flow diagram of the production of an incise drape for measurement.

[Figure 2] Figure 2 is a photograph taken when the adhesion force of the incise drape for measurement was measured.

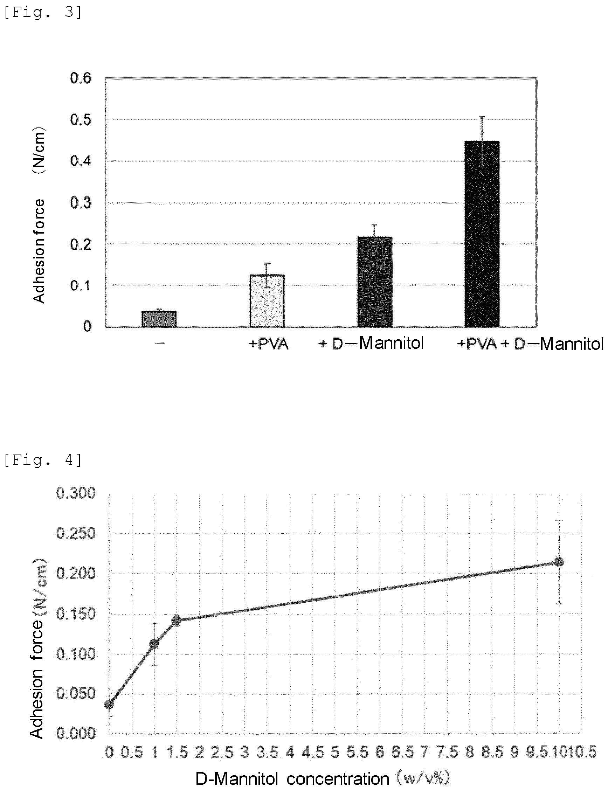

[Figure 3] Figure 3 is a diagram showing results (n = 3) of measuring the adhesion force (mean $\pm$ standard deviation) of the incise drape for measurement on artificial skin surfaces to which four types of test antiseptic solutions (a D-mannitol- and PVA-free test antiseptic solution [in the diagram, "-"] ; a test antiseptic solution comprising 0.5% PVA [in the diagram, "+PVA"]; a test antiseptic solution comprising 5.0% D-mannitol [in the diagram, "+D-mannitol"]; and a test antiseptic solution comprising 0.5% PVA and 5.0% D-mannitol [in the diagram, "+PVA+D-mannitol"]) were respectively applied, in Example 1.

[Figure 4] Figure 4 is a diagram showing results (n = 3) of measuring the adhesion force (mean $\pm$ standard deviation) of the incise drape for measurement on artificial skin surfaces to which test antiseptic solutions comprising varying concentrations (0%, 1.0%, 1.5%, or 10%) of D-mannitol were respectively applied, in Example 1.

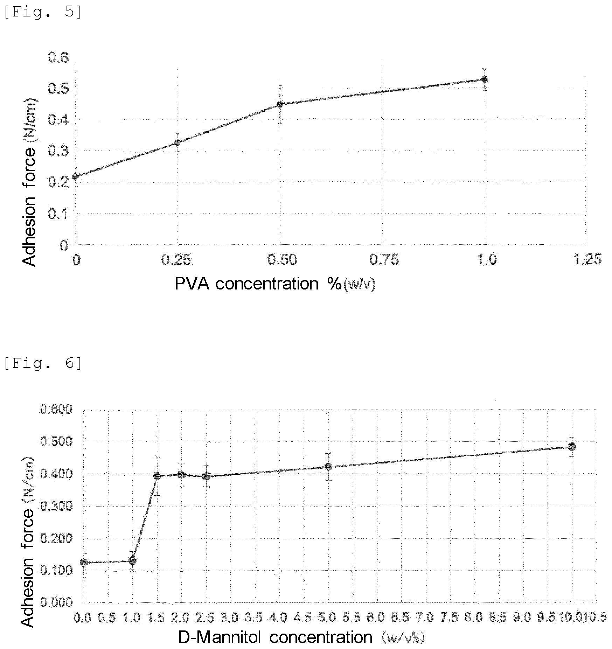

[Figure 5] Figure 5 is a diagram showing results (n = 3) of measuring the adhesion force (mean $\pm$ standard deviation) of the incise drape for measurement on artificial skin surfaces to which test antiseptic solutions comprising varying concentrations (0%, 0.25%, 0.5%, or 1.0%) of PVA and 5.0% D-mannitol were respectively applied, in Example 1.

[Figure 6] Figure 6 is a diagram showing results (n = 3) of measuring the adhesion force (mean $\pm$ standard deviation) of the incise drape for measurement on artificial skin surfaces to which test antiseptic solutions comprising 0.5% PVA and varying concentrations (0%, 1.0%, 1.5%, 2.0%, 2.5%, 5.0%, or 10%) of D-mannitol were respectively applied, in Example 1.

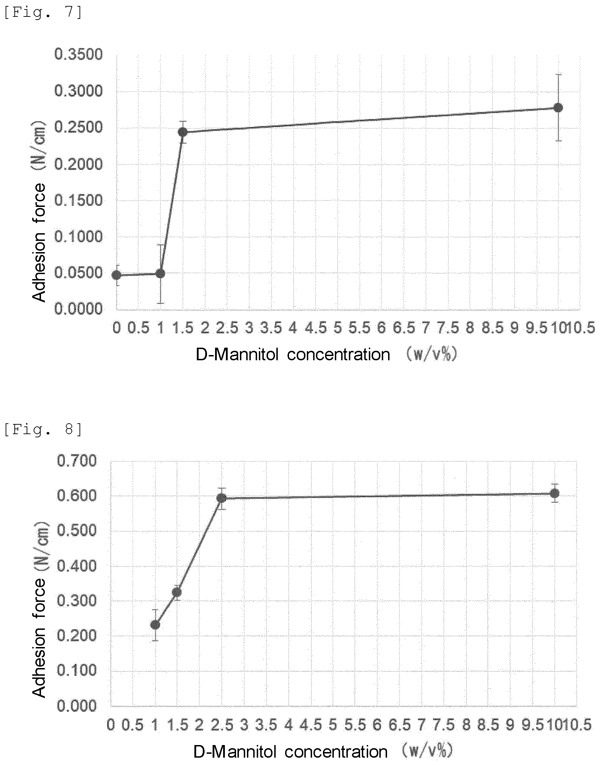

[Figure 7] Figure 7 is a diagram showing results (n = 3) of measuring the adhesion force (mean $\pm$ standard deviation) of the incise drape for measurement on artificial skin surfaces to which test antiseptic solutions comprising 0.1% PVA and varying concentrations (0%, 1.0%, 1.5%, or 10%) of D-mannitol were respectively applied, in Example 1.

[Figure 8] Figure 8 is a diagram showing results (n = 3) of measuring the adhesion force (mean $\pm$ standard deviation) of the incise drape for measurement on artificial skin surfaces to which test antiseptic solutions comprising 2.0% PVA and varying concentrations (1.0%, 1.5%, 2.5%, or 10%) of D-mannitol were respectively applied, in Example 1.

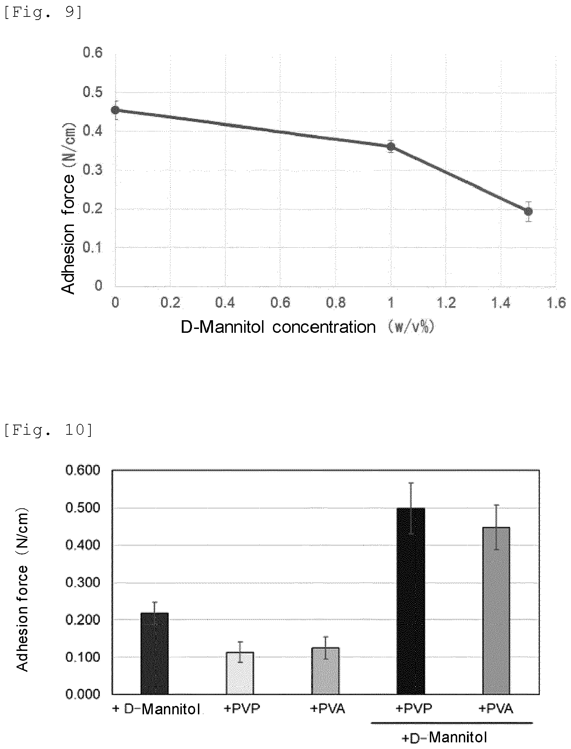

[Figure 9] Figure 9 is a diagram showing results (n = 3) of measuring the adhesion force (mean $\pm$ standard deviation) of the incise drape for measurement on artificial skin surfaces to which test antiseptic solutions comprising 3.5% PVA and varying concentrations (0%, 1.0%, or 1.5%) of D-mannitol were respectively applied, in Example 1.

[Figure 10] Figure 10 is a diagram showing results (n = 3) of measuring the adhesion force (mean $\pm$ standard deviation) of the incise drape for measurement on artificial skin surfaces to which five types of test antiseptic solutions (a test antiseptic solution comprising 5.0% D-mannitol [in the diagram, "+D-mannitol"]; a test antiseptic solution comprising 0.5% PVP [in the diagram, "+PVP"]; a test antiseptic solution comprising 0.5% PVA [in the diagram, "+PVA"]; a test antiseptic solution comprising 0.5% PVP and 5.0% D-mannitol [in the diagram, "+PVP+D-mannitol"]; and a test antiseptic solution comprising 0.5% PVA and 5.0% D-mannitol [in the diagram, "+PVA+D-mannitol"]) were respectively applied, in Example 1.

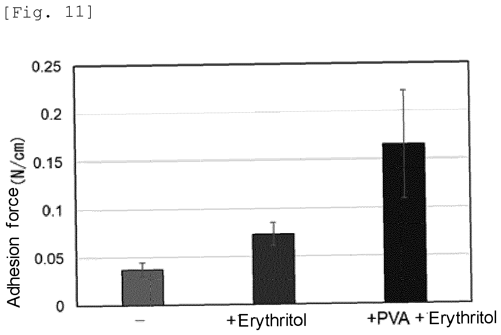

[Figure 11] Figure 11 is a diagram showing results (n = 3) of measuring the adhesion force (mean $\pm$ standard deviation) of the incise drape for measurement on artificial skin surfaces to which three types of test antiseptic solutions (an erythritol- and PVA-free test antiseptic solution [in the diagram, "-"]; a test antiseptic solution comprising 5.0% erythritol [in the diagram, "+erythritol"]; and a test antiseptic solution comprising 0.5% PVA and 5.0% erythritol [in the diagram, "+PVA+erythritol"]) were respectively applied, in Example 1.

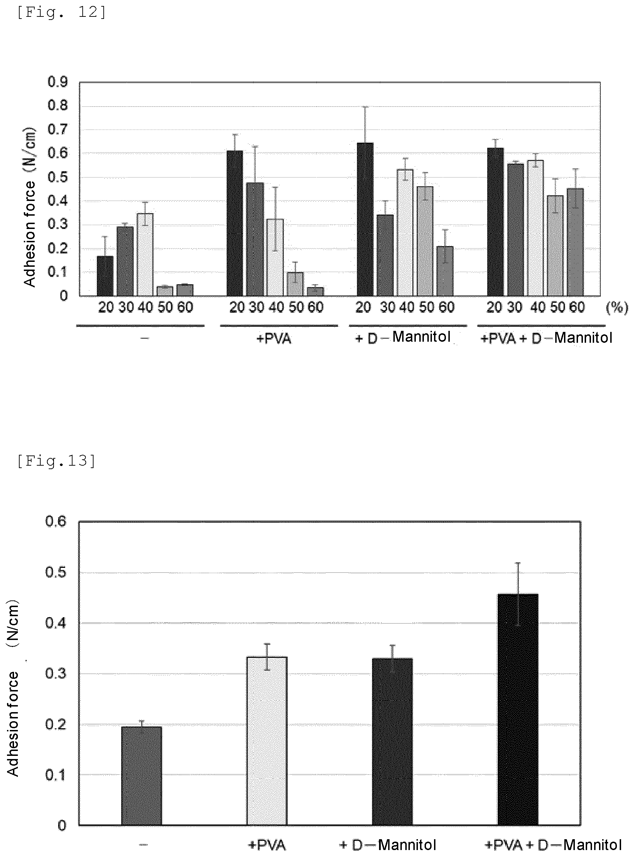

[Figure 12] Figure 12 is a diagram showing results (n = 3) of measuring the adhesion force (mean $\pm$ standard deviation) of the incise drape for measurement on artificial skin surfaces to which four types of test antiseptic solutions (a D-mannitol- and PVA-free test antiseptic solution [in the diagram, "-"]; a test antiseptic solution comprising 0.5% PVA [in the diagram, "+PVA"]; a test antiseptic solution comprising 5.0% D-mannitol [in the diagram, "+D-mannitol"]; and a test antiseptic solution comprising 0.5% PVA and 5.0% D-mannitol [in the diagram, "+PVA+D-mannitol"]) were respectively applied, under five types of humidity conditions (20%, 30%, 40%, 50%, and 60%), in Example 1.

[Figure 13] Figure 13 is a diagram showing results (n = 3) of measuring the adhesion force (mean $\pm$ standard deviation) of "Opsite Incise" on artificial skin surfaces to which four types of test antiseptic solutions (a D-mannitol- and PVA-free test antiseptic solution [in the diagram, "-"]; a test antiseptic solution comprising 0.5% PVA [in the diagram, "+PVA"]; a test antiseptic solution comprising 5.0% D-mannitol [in the diagram, "+D-mannitol"]; and a test antiseptic solution comprising 0.5% PVA and 5.0% D-mannitol [in the diagram, "+PVA+D-mannitol"]) were respectively applied, in Example 3.

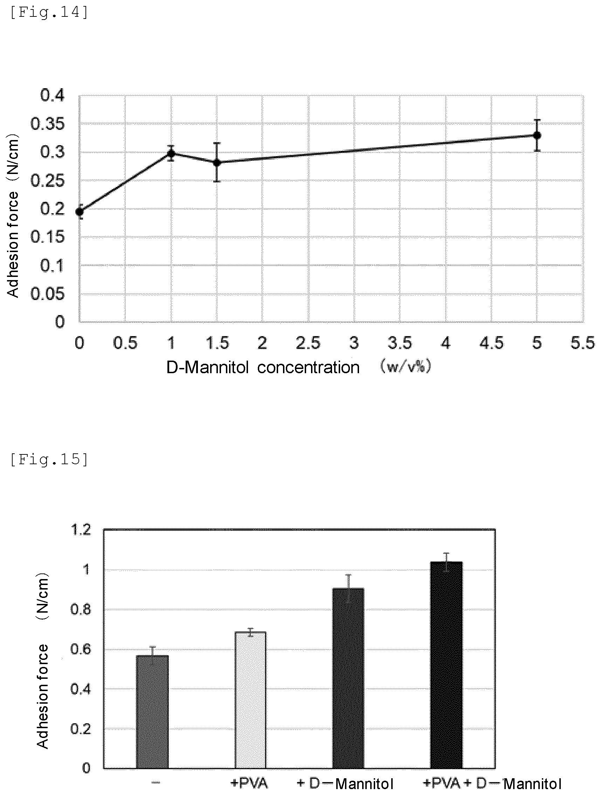

[Figure 14] Figure 14 is a diagram showing results (n = 3) of measuring the adhesion force (mean $\pm$ standard deviation) of "Opsite Incise" on artificial skin surfaces to which test antiseptic solutions comprising varying concentrations (0%, 1.0%, 1.5%, or 5%) of D-mannitol were respectively applied, in Example 3.

[Figure 15] Figure 15 is a diagram showing results (n = 3) of measuring the adhesion force (mean $\pm$ standard deviation) of "3M micropore S Kind Removal Silicone Tape" (corresponding to "3M™ Micropore™ S Surgical Tape" in the US, same in the following) on artificial skin surfaces to which four types of test antiseptic solutions (a D-mannitol- and PVA-free test antiseptic solution [in the diagram, "-"]; a test antiseptic solution comprising 0.5% PVA [in the diagram, "+PVA"]; a test antiseptic solution comprising 5.0% D-mannitol [in the diagram, "+D-mannitol"]; and a test antiseptic solution comprising 0.5% PVA and 5.0% D-mannitol [in the diagram, "+PVA+D-mannitol"]) were respectively applied in Example 3.

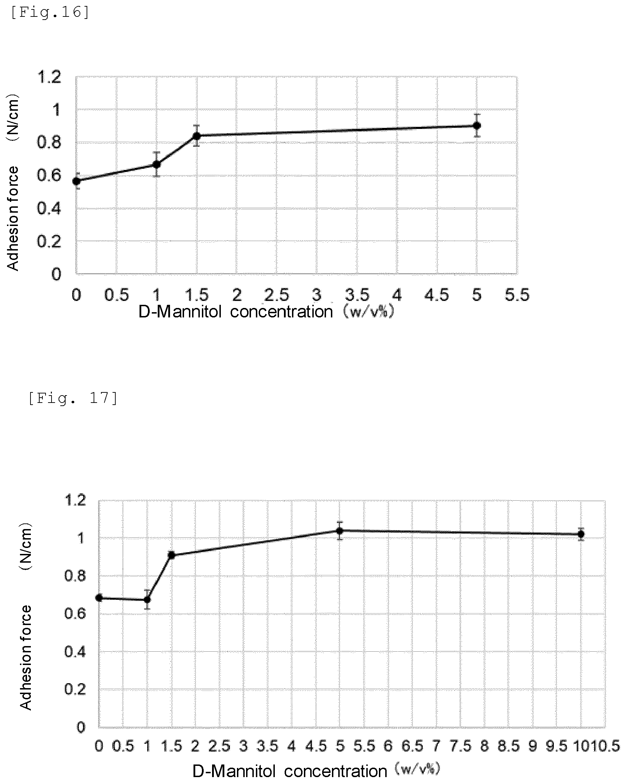

[Figure 16] Figure 16 is a diagram showing results (n = 3) of measuring the adhesion force (mean $\pm$ standard deviation) of "3M micropore S Kind Removal Silicone Tape" on artificial skin surfaces to which test antiseptic solutions comprising varying concentrations (0%, 1.0%, 1.5%, or 5%) of D-mannitol were respectively applied, in Example 3.

[Figure 17] Figure 17 is a diagram showing results (n = 3) of measuring the adhesion force (mean $\pm$ standard deviation) of "3M micropore S Kind Removal Silicone Tape" on artificial skin surfaces to which test antiseptic solutions comprising 0.5% PVA, and varying concentrations (0%, 1.0%, 1.5%, 5%, or 10%) of D-mannitol were respectively applied, in Example 3.

## Mode of Carrying Out the Invention

The present antiseptic composition is a composition comprising a microbicide and a sugar alcohol, intended for the purpose of "antisepsis". Owing to the effect of the sugar alcohol, the present antiseptic composition can reduce the risk of a medical tape such as incise drape coming off from the skin, without adversely affecting microbicidal activity, even when the medical tape such as incise drape is affixed to skin surface after application of the present antiseptic composition.

The microbicide can be a substance (compound) having a killing action on bacteria, fungi and/or viruses. Examples thereof can include olanexidine gluconate, benzalkonium salt such as benzalkonium chloride and benzalkonium alkyl phosphate, benzethonium chloride, triclosan, isopropyl methyl phenol, cetyl pyridinium chloride, resorcin, trichlorocarbanilide, chlorhexidine hydrochloride, chlorhexidine gluconate, polyhexamethylenebiguanide, sodium hypochlorite, hydrogen peroxide, povidone iodine, and iodine tincture. Olanexidine gluconate can be a preferred example because its effect has been demonstrated in the present examples mentioned later. These microbicides may each be blended singly into the present antiseptic composition or may be blended in combination of two or more thereinto. The concentration of the microbicide can be a concentration having sufficient microbicidal activity and is, for example, within the range of 0.01 to 20% (w/v), more preferably 0.1 to 10% (w/v), further preferably 1 to 5% (w/v).

The concentration of the sugar alcohol can be a concentration that can exert an action of enhancing the adhesion force of an incise drape on skin surface after application of the present antiseptic composition. Examples of the lower limit value of the concentration of the sugar alcohol can include 0.01% (w/v), 0.05% (w/v), 0.1% (w/v), 0.5% (w/v), 1.0% (w/v), 1.5% (w/v), 2.0% (w/v), and 2.5% (w/v). Examples of the upper limit value of the concentration of the sugar alcohol can include 50% (w/v), 40% (w/v), 30% (w/v), 25% (w/v), 20% (w/v), 15% (w/v), and 10% (w/v). Thus, examples of the concentration range of the sugar alcohol can include 0.01 to 50% (w/v), 0.05% (w/v) to 40% (w/v), 0.1 to 30% (w/v), 0.5 to 25% (w/v), 1.0 to 10% (w/v), 1.0 to 20% (w/v), 1.5 to 15% (w/v), 1.5 to 10% (w/v), 2.0 to 10% (w/v), and 2.5 to 10% (w/v). 1.0 to 10% (w/v) is preferred because its effect has been demonstrated in the present Examples mentioned later. For combined use with an adhesive agent, the concentration range is preferably 1.0 to 10% (w/v), more preferably 1.5 to 10% (w/v), further preferably 2.5 to 10% (w/v).

The sugar alcohol is characterized by having an action of enhancing the adhesion force of an incise drape on skin surface after application of the present antiseptic composition. The sugar alcohol can be a sugar that is formed by the reduction of a carboxyl group in aldose or ketose. Examples thereof can include erythritol, threitol, ribitol, arabitol, xylitol, sorbitol, galactitol, iditol, allitol, altritol, lactitol, maltitol, mannitol, and sucrose. The sugar alcohol may be an $\alpha$ form, may be a $\beta$ form, and may be any of D and L forms or (+) and (-) forms without particular limitations. Alternatively, a racemate may be used. D-Mannitol or erythritol can be a preferred example of the sugar alcohol because its effect has been demonstrated in the present Examples mentioned later. These sugar alcohols may each be blended alone into the present antiseptic composition or may be blended in combination of two or more thereinto.

The sugar alcohol may exhibit a high critical relative humidity (CRH) (i.e., low moisture absorbency). Examples of the CRH of the sugar alcohol can include 55% or more, 60% or more, 65% or more, 70% or more, 75% or more, 80% or more, 85% or more, 86% or more, 87% or more, 88% or more, 89% or more, 90% or more, 91% or more, 92% or more, 93% or more, 94% or more, 95% or more, 96% or more, 97% or more, and 98% or more at 25°C. The CRH can be measured by a routine method.

In the present specification, the "clinical relative humidity" means a relative humidity at which the weight of the sugar alcohol starts to increase rapidly when its relative humidity is elevated at a constant temperature (e.g., 25°C).

The present antiseptic composition preferably further comprises an adhesive agent for further enhancing the adhesion force of an incise drape on skin surface after application of the present antiseptic composition, and/or for increasing microbicidal activity on viable microbes such as *Candida albicans*.

The lower limit value of the concentration of the adhesive agent can be a concentration that can further enhance the adhesion force of an incise drape on skin surface after application of the present antiseptic composition, and/or increase microbicidal activity on viable microbes such as *Candida albicans*. Examples thereof can include 0.01% (w/v), 0.03% (w/v), 0.06% (w/v), 0.1% (w/v), 0.2% (w/v), 0.25% (w/v), 0.5% (w/v), 1.0% (w/v), 1.3% (w/v), and 1.6% (w/v). On the other hand, the upper limit value of the concentration of the adhesive agent is preferably less than 3.5% (w/v) (e.g., 3.3% (w/v) or less, 3.0% (w/v) or less, 2.6% (w/v) or less, 2.3% (w/v) or less, 2.0% (w/v) or less, 1.6% (w/v) or less, 1.3% (w/v) or less, or 1.0% (w/v) or less), more preferably 2.0% (w/v) or less, from the viewpoint of avoiding a high concentration because 1) the present antiseptic composition (comprising a sugar alcohol) supplemented with a high concentration of the adhesive agent might rather reduce the adhesion force of an incise drape on skin surface after being applied to the skin surface, 2) as a result of drying the present antiseptic composition comprising a high concentration of the adhesive agent to prepare a thick film on skin surface, the film of the antiseptic composition exhibits reduced the adhesion force on the skin and might come off partially or wholly, 3) no adhesion force to the skin exists in a portion where the film of the antiseptic composition has come off, and particularly, when the film of the antiseptic composition has come off wholly, the overall the adhesion force of an incise drape to the skin might be reduced, and 4) the present antiseptic composition comprising a high concentration of the adhesive agent might have poor applicability depending on a purpose as a result of elevation in viscosity. Thus, examples of the concentration range of the adhesive agent can include 0.01 to less than 3.5% (w/v) (e.g., 3.3% (w/v) or less, 3.0% (w/v) or less, 2.6% (w/v) or less, 2.3% (w/v) or less, 2.0% (w/v) or less, 1.6% (w/v) or less, 1.3% (w/v) or less, or 1.0% (w/v) or less), 0.03 to less than 3.5% (w/v) (e.g., 3.3% (w/v) or less, 3.0% (w/v) or less, 2.6% (w/v) or less, 2.3% (w/v) or less, 2.0% (w/v) or less, 1.6% (w/v) or less, 1.3% (w/v) or less, or 1.0% (w/v) or less), 0.06 to less than 3.5% (w/v) (e.g., 3.3% (w/v) or less, 3.0% (w/v) or less, 2.6% (w/v) or less, 2.3% (w/v) or less, 2.0% (w/v) or less, 1.6% (w/v) or less, 1.3% (w/v) or less, or 1.0% (w/v) or less), 0.1 to less than 3.5% (w/v) (e.g., 3.3% (w/v) or less, 3.0% (w/v) or less, 2.6% (w/v) or less, 2.3% (w/v) or less, 2.0% (w/v) or less, 1.6% (w/v) or less, 1.3% (w/v) or less, or 1.0% (w/v) or less), 0.2 to less than 3.5% (w/v) (e.g., 3.3% (w/v) or less, 3.0% (w/v) or less, 2.6% (w/v) or less, 2.3% (w/v) or less, 2.0% (w/v) or less, 1.6% (w/v) or less, 1.3% (w/v) or less, or 1.0% (w/v) or less), 0.25 to less than 3.5% (w/v) (e.g., 3.3% (w/v) or less, 3.0% (w/v) or less, 2.6% (w/v) or less, 2.3% (w/v) or less, 2.0% (w/v) or less, 1.6% (w/v) or less, 1.3% (w/v) or less, or 1.0% (w/v) or less), 0.5% (w/v) to less than 3.5% (w/v) (e.g., 3.3% (w/v) or less, 3.0% (w/v) or less, 2.6% (w/v) or less, 2.3% (w/v) or less, 2.0% (w/v) or less, 1.6% (w/v) or less, 1.3% (w/v) or less, or 1.0% (w/v) or less), 1.0% (w/v) to less than 3.5% (w/v) (e.g., 3.3% (w/v) or less, 3.0% (w/v) or less, 2.6% (w/v) or less, 2.3% (w/v) or less, 2.0% (w/v) or less, 1.6% (w/v) or less, or 1.3% (w/v) or less), 1.3% (w/v) to less than 3.5% (w/v) (e.g., 3.3% (w/v) or less, 3.0% (w/v) or less, 2.6% (w/v) or less, 2.3% (w/v) or less, 2.0% (w/v) or less, or 1.6% (w/v) or less), and 1.6% (w/v) to less than 3.5% (w/v) (e.g., 3.3% (w/v) or less, 3.0% (w/v) or less, 2.6% (w/v) or less, 2.3% (w/v) or less, or 2.0% (w/v) or less).

The adhesive agent is characterized by having an action of enhancing the adhesion force of an incise drape on skin surface after application of the present antiseptic composition. Examples of the adhesive agent can include a vinyl polymer compound, an acrylic polymer compound, and a cellulose polymer compound.

In the present specification, the "vinyl polymer compound" means a polymer (vinyl polymer) that is obtained by polymerizing a monomer compound having a vinyl group, followed by saponification, if necessary. Examples of the vinyl polymer compound can include polyvinyl alcohol (PVA) (complete or partial saponification product), polyvinylpyrrolidone (PVP), polyvinyl acetate, polyethylene, polypropylene, polystyrene, polyvinyl chloride, polymethyl methacrylate, polyacrylic acid, a carboxyvinyl polymer, polyvinylacetal, polyvinylidene chloride, and a PVA/PVP copolymer. PVA or PVP can be a preferred example because its effect has been demonstrated in the present Examples mentioned later.

The molecular weight of the vinyl polymer compound differs depending on the type of a constituent monomer, etc. and therefore, cannot be generalized. The weight-average molecular weight is usually 1000 to 2500000, preferably 5000 to 2000000, further preferably 10000 to 1500000. More specifically, when the vinyl polymer compound is polyvinyl alcohol, the average degree of polymerization of the polyvinyl alcohol measured in accordance with JIS K-6726 is usually within the range of 100 to 4000, preferably 200 to 3000, more preferably 300 to 2000. When the vinyl polymer compound is polyvinylpyrrolidone, the K value [viscosity characteristic value] of the polyvinylpyrrolidone by the Fikentscher method is usually 10 to 120, preferably 30 to 110, more preferably 60 to 100, further preferably 80 to 100.

In the present specification, the "acrylic polymer compound" means a polymerization product comprising a monomer unit derived from a (meth)acrylic monomer in a polymer structure, and typically means a polymerization product comprising a monomer unit derived from a (meth)acrylic monomer at a proportion of more than 50% by weight. Examples of the acrylic polymer compound can include polyacrylic acid, sodium polyacrylate, a carboxyvinyl polymer, polyacrylamide, and a polyacrylamide/acrylate copolymer.

In the present specification, the "cellulose polymer compound" means cellulose (cellulose polymer) in which some or all hydroxyl groups of cellulose are substituted. Examples of the cellulose polymer compound can include ethylcellulose, carboxymethylcellulose, sodium carboxymethylcellulose, hydroxyethylcellulose, hydroxypropylcellulose, methylcellulose, nitrocellulose, and cationized cellulose. The adhesive agents described above may each be blended singly into the present antiseptic composition or may be blended in combination of two or more thereinto.

Examples of the lower limit value of the weight ratio between the adhesive agent and the sugar alcohol in the present antiseptic composition further comprising the adhesive agent can include 1:0.5, 1:0.75, 1:1, 1:1.2, 1:1.25, 1:1.5, 1:2, 1:2.5, and 1:3.0. Examples of the upper limit value of the weight ratio between the adhesive agent and the sugar alcohol in the present antiseptic composition further comprising the adhesive agent can include 1:40, 1:36, 1:33, 1:30, 1:26, 1:23, 1:20, 1:16, 1:13, and 1:10. Thus, examples of the range of the weight ratio between the adhesive agent and the sugar alcohol in the present antiseptic composition further comprising the adhesive agent can include 1:0.5 to 1:40, 1:0.5 to 1:36, 1:0.5 to 1:33, 1:0.5 to 1:30, 1:0.5 to 1:26, 1:0.5 to 1:23, 1:0.5 to 1:20, 1:0.5 to 1:16, 1:0.5 to 1:13, 1:0.5 to 1:10, 1:0.75 to 1:40, 1:0.75 to 1:36, 1:0.75 to 1:33, 1:0.75 to 1:30, 1:0.75 to 1:26, 1:0.75 to 1:23, 1:0.75 to 1:20, 1:0.75 to 1:16, 1:0.75 to 1:13, 1:0.75 to 1:10, 1:1 to 1:40, 1:1 to 1:36, 1:1 to 1:33, 1:1 to 1:30, 1:1 to 1:26, 1:1 to 1:23, 1:1 to 1:20, 1:1 to 1:16, 1:1 to 1:13, 1:1 to 1:10, 1:1.2 to 1:40, 1:1.2 to 1:36, 1:1.2 to 1:33, 1:1.2 to 1:30, 1:1.2 to 1:26, 1:1.2 to 1:23, 1:1.2 to 1:20, 1:1.2 to 1:16, 1:1.2 to 1:13, 1:1.2 to 1:10, 1:1.25 to 1:40, 1:1.25 to 1:36, 1:1.25 to 1:33, 1:1.25 to 1:30, 1:1.25 to 1:26, 1:1.25 to 1:23, 1:1.25 to 1:20, 1:1.25 to 1:16, 1:1.25 to 1:13, 1:1.25 to 1:10, 1:1.5 to 1:40, 1:1.5 to 1:36, 1:1.5 to 1:33, 1:1.5 to 1:30, 1:1.5 to 1:26, 1:1.5 to 1:23, 1:1.5 to 1:20, 1:1.5 to 1:16, 1:1.5 to 1:13, 1:1.5 to 1:10, 1:2 to 1:40, 1:2 to 1:36, 1:2 to 1:33, 1:2 to 1:30, 1:2 to 1:26, 1:2 to 1:23, 1:2 to 1:20, 1:2 to 1:16, 1:2 to 1:13, 1:2 to 1:10, 1:2.5 to 1:40, 1:2.5 to 1:36, 1:2.5 to 1:33, 1:2.5 to 1:30, 1:2.5 to 1:26, 1:2.5 to 1:23, 1:2.5 to 1:20, 1:2.5 to 1:16, 1:2.5 to 1:13, 1:2.5 to 1:10, 1:3 to 1:40, 1:3 to 1:36, 1:3 to 1:33, 1:3 to 1:30, 1:3 to 1:26, 1:3 to 1:23, 1:3 to 1:20, 1:3 to 1:16, 1:3 to 1:13, and 1:3 to 1:10.

In the present specification, the "incise drape" means a film drape for incision that is used for the purpose of preventing SSI (e.g., infection by skin resident microbiota, bacteria, fungi, virus, and the like) when affixed to the skin of a patient, followed by surgery (incision operation) thereon. The incise drape is usually water-proof, well fits in the body shape of a patient, has moderate water vapor permeability, and contains an adhesive agent applied thereon. The incise drape is commercially available and includes, for example, "3M(R) Steri-Drape(R)", "3M loban(R) Special Incise Drape"(all manufactured by 3M), and "Opsite (R) Incise" (manufactured by Smith & Nephew).

The present antiseptic composition may further comprise an optional component in addition to the microbicide and the sugar alcohol. In the present specification, examples of the "optional component" can include a tonicity agent (e.g., glucose, sorbitol, mannitol, lactose, and sodium chloride), a chelating agent (e.g., EDTA, EGTA, citric acid, and salicylate), a solubilizing agent, a colorant, a preservative, an antioxidant, an amino acid (e.g., proline and glutamine), a buffer (e.g., CAPS, Bicine, Gricine, Tricine, Tris, HEPPS, TAPS, and Bicine), a phospholipid (e.g., lysophosphatidic acid [LPA]), and a pH adjuster (e.g., glucono-5-lactone, sodium hydroxide, and sodium bicarbonate). In the present specification, the "optional component" means a component that may or may not be contained.

The present antiseptic composition may comprise a colorant in order to identify a location where the present antiseptic composition has been applied. In this case, the present antiseptic composition usually further comprises a solubilizing agent in order to prevent an insoluble salt from being formed through the reaction of the colorant with the microbicide.

Examples of the solubilizing agent can include a surfactant (a nonionic surfactant and an ionic surfactant), ethylenediamine, sodium benzoate, nicotinic acid amide, cyclodextrin, ethanol, benzyl alcohol, and propylene glycol. Preferred examples of the ionic surfactant can include alkyl dimethylamine oxide such as oleyl dimethylamine oxide, stearyl dimethylamine oxide, palmityl dimethylamine oxide, myristyl dimethylamine oxide, lauryl dimethylamine oxide, and coconut oil alkyl dimethylamine oxide. Among them, lauryl dimethylamine oxide is preferred. Examples of the nonionic surfactant can include sorbitan fatty acid ester, polyoxyethylene sorbitan fatty acid ester, polyoxyethylene alkyl ether, polyoxyethylene polyoxypropylene alkyl ether, polyoxyethylene polyoxypropylene glycol, polyglycerin fatty acid ester, polyoxyethylene hydrogenated castor oil, and sucrose fatty acid ester. Among them, polyoxyethylene polyoxypropylene glycol, polyoxyethylene alkyl ether, or polyoxyethylene polyoxypropylene alkyl ether is preferred. Examples of the polyoxyethylene polyoxypropylene glycol can include polyoxyethylene (42) polyoxypropylene (67) glycol (Pluronic P-123), polyoxyethylene (54) polyoxypropylene (39) glycol (Pluronic P-85), polyoxyethylene (196) polyoxypropylene (67) glycol (Pluronic F-127), polyoxyethylene (42) polyoxypropylene (67) glycol (Pluronic P-123), polyoxyethylene (3) polyoxypropylene (17) glycol (Pluronic L-31), polyoxyethylene (20) polyoxypropylene (20) glycol (Pluronic L-44), polyoxyethylene (120) polyoxypropylene (40) glycol (Pluronic F-87), and polyoxyethylene (160) polyoxypropylene (30) glycol (Pluronic F-68). Among them, polyoxyethylene (20) polyoxypropylene (20) glycol (Pluronic L-44) is preferred. Examples of the polyoxyethylene alkyl ether can include polyoxyethylene cetyl ether, polyoxyethylene oleyl ether, and polyoxyethylene lauryl ether (lauromacrogol). Particularly, polyoxyethylene lauryl ether (lauromacrogol) is preferred. Examples of the polyoxyethylene polyoxypropylene alkyl ether can include polyoxyethylene (20) polyoxypropylene (4) cetyl ether, polyoxyethylene (30) polyoxypropylene (6) decyl tetradecyl ether, and polyoxyethylene (25) polyoxypropylene (25) lauryl ether. Particularly, polyoxyethylene (20) polyoxypropylene (4) cetyl ether is preferred. These solubilizing agents may each be blended singly into the present antiseptic composition or may be blended in combination of two or more thereinto. The concentration of the solubilizing agent can be a concentration that prevents the precipitation of the microbicide and does not reduce microbicidal activity. The solubilizing agent is usually used at a concentration of 0.1 to 30% (w/v), preferably 0.5 to 15% (w/v), more preferably 1 to 8% (w/v).

The colorant can be a coloring material that is not toxic to living bodies. Examples thereof can include a tar dye (e.g., Red No. 2 [Amaranth], Red No. 3 [Erythrocin], Red No. 102 [New Coccine], Red No. 201 [Lithol Rubine B], Yellow No. 4 [Tartrazine], Yellow No. 5 [Sunset Yellow], Green No. 3 [Fast Green], Blue No. 1 [Brilliant Blue], Blue No. 2 [Indigo Carmine], Blue No. 205 [Alphazurine FG], Blue No. 403 [Sudan Blue B], Violet No. 201 [Alizurine Purple SS], and Violet No. 401 [Alizurol Purple]). The concentration of the colorant is usually within the range of 0.001 to 1.0% (w/v), preferably 0.005 to 0.5% (w/v).

The present antiseptic composition may be a non-liquid type and is preferably a liquid type (antiseptic solution). A non-liquid type of the present antiseptic composition, such as a powder, comprising the microbicide and the sugar alcohol can be dissolved in a solvent to prepare a liquid type of the present antiseptic composition. In the present specification, examples of the "solvent" can include deionized water, distilled water, saline, PBS (phosphate buffered saline), and TBS (Tris buffered saline). When the present antiseptic composition is a non-liquid type, the concentrations of the microbicide, the sugar alcohol, and the adhesive agent in the present antiseptic composition are the concentrations of the microbicide, the sugar alcohol, and the adhesive agent in a liquid type of the present antiseptic composition prepared by dissolving the non-liquid type of the present antiseptic composition in a solvent, and the weight ratio between the adhesive agent and the sugar alcohol in the present antiseptic composition is the weight ratio between the adhesive agent and the sugar alcohol in a liquid type of the present antiseptic composition prepared by dissolving the non-liquid type of the present antiseptic composition in a solvent.

The subject to which the present antiseptic composition is to be applied can be a subject in need of antisepsis. Examples thereof can include: a subject (patient) having a wound; such a subject having a wound, the subject being in need of affixing a transparent dressing (transparent wound dressing material) to a site where a liquid type of the present antiseptic composition has been applied; a subject (patient) in need of surgery (e.g., cranial nerve surgery, cardiovascular surgery, gastroenterological [e.g., gastric, large intestinal, hepatic, or pancreatic] surgery, or cosmetic plastic surgery) ; and such a subject in need of surgery, the subject being in need of affixing an incise drape to a site where a liquid type of the present antiseptic composition has been applied. The subject is preferably such a subject in need of surgery, the subject being in need of affixing an incise drape to a site where a liquid type of the present antiseptic composition has been applied.

The application site of the present antiseptic composition is, for example, a wound site when the subject to which the present antiseptic composition is to be applied is a subject having a wound, or an operation site (usually, the skin) when the subject is a subject in need of surgery. When the application site of the present antiseptic composition is the skin (e.g., skin surface or subcutaneous tissue), the method for applying the present antiseptic composition is usually application. Examples of the method for applying the present antiseptic composition to the skin of the subject can include an application method using a base material, such as paper, cloth, nonwoven fabric, cotton swab, or adsorbent cotton, impregnated with a liquid type of the present antiseptic composition, an application method using an applicator for application filled with a liquid type of the present antiseptic composition, and an application method using a liquid type of the present antiseptic composition as a rubbing agent or a scrub agent.

Conditions, such as temperature and humidity, when the present antiseptic composition is applied are not particularly limited. The temperature is, for example, room temperature (e.g., within the range of 15 to 35°C, preferably 15 to 30°C, more preferably 18 to 28°C, further preferably 20 to 26°C), and the humidity is, for example, within the range of 10 to 90%, preferably 20 to 80%, more preferably 30 to 70%, further preferably 40 to 60%.

Hereinafter, the present invention will be described more specifically with reference to Examples. However, the technical scope of the present invention is not limited by these examples.

**Examples**

Example 1. Measurement of adhesion force of incise drape on skin surface to which present antiseptic composition was applied (1)

1. Material and method

1-1 Test antiseptic solution

The test antiseptic solutions used were Olanedine antiseptic solution (1) shown in Table 1 below, supplemented with varying concentrations of PVA (Gohsenol EG-05P, average degree of polymerization: approximately 500, partial saponification product, manufactured by Mitsubishi Chemical Corp.) or PVP (polyvinylpyrrolidone K90, K value: 90, manufactured by FUJIFILM Wako Pure Chemical Corp.) and/or varying concentrations of D-mannitol (PEARLITOL 160C, manufactured by Roquette Freres) or erythritol (mesoerythritol, manufactured by FUJIFILM Wako Pure Chemical Corp.) .

[Table 1]

| Components of Olanedine antiseptic solution (1) and their quantities | |
| --- | --- |
| Component | Quantity (concentration) |
| Olanexidine gluconate (active ingredient) | 1.5% |
| Polyoxyethylene (20) polyoxypropylene (20) glycol (solubilizing agent) | 1.08% |
| Lauromacrogol (solubilizing agent) | 2.0% |
| Lauryl dimethylamine oxide (solubilizing agent) | 2.5% |
| Yellow No. 5 (Sunset Yellow) (colorant) | 0.1% |
| Glucono-$\delta$-lactone (pH adjuster) | q.s. |
| Sodium hydroxide (pH adjuster) | q.s. |
| Purified water | q.s. |

The pH adjusters in the table were used for adjusting the pH of the test antiseptic solution to 4 to 7.

1-2 Preparation of incise drape for measurement

3M Ioban Special Incise Drape (total size: 15 cm × 20 cm, adhesive agent portion size: 10 cm × 20 cm) (6035, manufactured by 3M Company) was cut into a width of 4 cm (total size: 15 cm × 4 cm, adhesive agent portion size: 10 cm × 4 cm). Curing tapes were affixed to both ends on the long sides, and the cut piece was folded back such that the size of the adhesive agent portion was 4 cm × 4 to 5 cm (approximately 10 cm and approximately 3 cm knobs were attached thereto). The approximately 10 cm knob was used as a gripping margin to prepare an incise drape for measurement (see Figure 1).

1-3 Measurement of adhesion force of incise drape for measurement

The adhesion force of the incise drape for measurement on an artificial skin surface to which each test antiseptic solution was applied was measured according to the following procedures [1] to [7] (see Figure 2).

[1] 100 μL of the test antiseptic solution was added dropwise to the surface of an artificial skin (Bioskin Plate (50 mm square), part number: No. 64, size: 50 mm × 50 mm × 5T mm, manufactured by Beaulax Co., Ltd.), spread throughout the artificial skin surface using a platinum loop, and dried in air at room temperature (23 ± 3°C) for approximately 30 minutes.
[2] The incise drape for measurement was lightly affixed to the surface of the artificial skin to which the test antiseptic solution was applied, and then pressure-bonded thereto using a manual pressure bonding apparatus (pressure bonding roller for peeling test) (manufactured by Imada Co., Ltd.) by two round trips at a rate of approximately 10 mm per second. In this process, it was confirmed that: the manual pressure bonding apparatus was in no contact with the adhesive surface of the incise drape for measurement; the incise drape had no wrinkle; and no air was involved between the artificial skin and the incise drape for measurement. After the pressure bonding, the resultant was left standing at room temperature.
[3] Approximately 30 minutes later, the gripping margin was folded back 180° such that the portions on the back side of the incise drape for measurement overlapped with each other. The artificial skin was mounted to the upper flat chuck of measurement equipment (digital force gauge system [prepared by attaching a digital force gauge (model: ZTS-5N) to a vertical electric measurement stand (model: MX2-500N), connecting the resultant to a computer, and attaching a flat chuck (model: GC-1200) to the measurement axis of the digital force gauge]) (manufactured by Imada Co., Ltd.).
[4] The load of the measurement equipment was reset to zero in a floating state of the artificial skin and the incise drape for measurement.
[5] The gripping margin of the incise drape for measurement was vertically put down and mounted to the lower part of the measurement equipment.
[6] The artificial skin was uplifted at 100 mm/sec so that the incise drape for measurement was peeled 180°. The load at the time of peeling was measured and recorded using Force Recorder Standard (load-time graph creation software) (manufactured by Imada Co., Ltd.). The recording rate was set to 0.005 seconds. The measurement was terminated when the incise drape for measurement was completely peeled from the artificial skin.

[7] The adhesion force per cm in width of the incise drape for measurement (N/cm) was determined on an artificial skin basis. The adhesion force was defined as an average load from points 1 to 2 when the point in time (sec) of 25% after the start of measurement was defined as point 1 and the subsequent point in time (sec) of 50% of the peeled length was defined as point 2 (see the document "Dai-17 Kaisei Nippon Yakkyokuho Kaisetsu Sho (Description of Japanese Pharmacopoeia 17th edition in English), testing method for the adhesion force. Hirokawa Shoten (Tokyo, Japan); 2016. p. B-664-71"). The values of point 1 (= total load measurement time $\times$ 0.25) and point 2 (point 1 + [total load measurement time - point 1] $\times$ 0.5) calculated to the tenths-place digit were input in the Force Recorder Standard to calculate the adhesion force. The total load measurement time was calculated according to recording rate (0.005 sec) $\times$ the number of data. The present antiseptic composition is supposed to be used in an operating room, and the humidity of the operating room is usually controlled around 50%. Hence, the adhesion force measurement was performed at a humidity of 50 (% RH) except for the section "2-7" given below. The adhesion force differs largely among tests, and the adhesion force cannot be properly compared among different tests. Therefore, the adhesion force was compared among the same tests.

2. Results

2-1 Effect of sugar alcohol in present antiseptic composition, and effect of present antiseptic composition supplemented with adhesive agent

In order to confirm the effect of a sugar alcohol in the present antiseptic composition, and the effect of the present antiseptic composition supplemented with an adhesive agent, the adhesion force of the incise drape for measurement was measured using four types of test antiseptic solutions shown in Table 2.

As a result, when the incise drape for measurement was affixed to an artificial skin to which the test antiseptic solution comprising D-mannitol was applied, the enhanced the adhesion force of the incise drape for measurement was shown as compared with the case where the incise drape for measurement was affixed to an artificial skin to which the test antiseptic solution free of both D-mannitol and PVA or the test antiseptic solution comprising PVA was applied (see Table 2 and Figure 3).

This result indicates that the present antiseptic composition (i.e., the antiseptic composition comprising the sugar alcohol [D-mannitol]) increases the adhesion force of an incise drape on skin surface to which the antiseptic composition has been applied, as compared with a sugar alcohol (D-mannitol)-free antiseptic composition or an antiseptic composition comprising an adhesive agent (PVA).

When the incise drape for measurement was affixed to an artificial skin to which the test antiseptic solution comprising both PVA and D-mannitol was applied, the synergistically enhanced the adhesion force of the incise drape for measurement was shown as compared with the case where the incise drape for measurement was affixed to an artificial skin to which the test antiseptic solution comprising PVA or D-mannitol alone was applied (see Table 2 and Figure 3).

This result indicates that the present antiseptic composition (i.e., the antiseptic composition comprising the sugar alcohol [D-mannitol]) supplemented with the adhesive agent (PVA) synergistically enhances the adhesion force of an incise drape on skin surface to which the antiseptic composition has been applied, as compared with an antiseptic composition comprising the sugar alcohol (D-mannitol) or the adhesive agent (PVA) alone.

[Table 2]

| Test antiseptic solution | Adhesion force (N/cm) |
|---|---|
| D-mannitol- and pva-free test antiseptic solution | 0.037 $\pm$ 0.007 |
| Test antiseptic solution comprising 0.5% PVA | 0.124 $\pm$ 0.030 |
| Test antiseptic solution comprising 5% D-mannitol | 0.217 $\pm$ 0.030 |
| Test antiseptic solution comprising 0.5% PVA and 5% D-mannitol | 0.447 $\pm$ 0.060 |

Figure 3 was created on the basis of the results of Table 2.

2-2 Study on concentration of sugar alcohol in present antiseptic composition

In order to study the concentration of a sugar alcohol in the present antiseptic composition, the adhesion force of the incise drape for measurement was measured using four types of test antiseptic solutions shown in Table 3.

As a result, when the incise drape for measurement was affixed to an artificial skin to which the test antiseptic solution comprising 1.0 to 10% D-mannitol was applied, the enhanced the adhesion force of the incise drape for measurement was shown as compared with the case where the incise drape for measurement was affixed to an artificial skin to which the D-mannitol-free test antiseptic solution was applied (see Table 3 and Figure 4).

This result indicates that the present antiseptic composition having a sugar alcohol (D-mannitol) concentration adjusted to at least 1.0 to 10% enhances the adhesion force of an incise drape on skin surface to which the antiseptic composition has been applied, as compared with the case of using a sugar alcohol (D-mannitol)-free antiseptic composition.

[Table 3]

| Test antiseptic solution | Adhesion force (N/cm) |
|---|---|
| D-mannitol-free test antiseptic solution | 0.037 ± 0.015 |
| Test antiseptic solution comprising 1% D-mannitol | 0.112 ± 0.026 |
| Test antiseptic solution comprising 1.5% D-mannitol | 0.142 ± 0.007 |
| Test antiseptic solution comprising 10% D-mannitol | 0.214 ± 0.052 |

Figure 4 was created on the basis of the results of Table 3.

2-3 Study on concentration of adhesive agent to be added to present antiseptic composition

In order to study the concentration of an adhesive agent to be added to the present antiseptic composition, the adhesion force of the incise drape for measurement was measured using four types of test antiseptic solutions shown in Table 4.

As a result, when the incise drape for measurement was affixed to an artificial skin to which the test antiseptic solution comprising 0.25 to 1.0% PVA and 5% D-mannitol was applied, the enhanced the adhesion force of the incise drape for measurement was shown as compared with the case where the incise drape for measurement was affixed to an artificial skin to which the test antiseptic solution comprising only 5% D-mannitol was applied (see Table 4 and Figure 5).

This result indicates that the addition of an adhesive agent (PVA), particularly, 0.25 to 1.0% of the adhesive agent, to the present antiseptic composition (i.e., the antiseptic composition comprising the sugar alcohol [D-mannitol]) can further enhance the adhesion force of an incise drape on skin surface to which the antiseptic composition has been applied, as compared with the present antiseptic composition free of the adhesive agent (PVA). 0.25 to 1.0% of the adhesive agent (PVA) did not cause a serious problem such as coming off of a film.

[Table 4]

| Test antiseptic solution | Adhesion force (N/cm) |
|---|---|
| Test antiseptic solution comprising 5% D-mannitol | 0.217 ± 0.076 |
| Test antiseptic solution comprising 0.25% PVA and 5% D-mannitol | 0.325 ± 0.012 |
| Test antiseptic solution comprising 0.5% PVA and 5% D-mannitol | 0.447 ± 0.065 |
| Test antiseptic solution comprising 1% PVA and 5% D-mannitol | 0.526 ± 0.125 |

Figure 5 was created on the basis of the results of Table 4.

2-4 Study on concentration of sugar alcohol in present antiseptic composition supplemented with adhesive agent

In order to study the concentration of a sugar alcohol in the present antiseptic composition supplemented with an adhesive agent, the adhesion force of the incise drape for measurement was measured using seven types of test antiseptic solutions shown in Table 5.

As a result, by affixing the incise drape for measurement to an artificial skin to which the test antiseptic solution comprising 0.5% PVA and 1.0 to 10.0% D-mannitol was applied, the enhanced the adhesion force was shown. Particularly, when the incise drape for measurement was affixed to an artificial skin to which the test antiseptic solution comprising 0.5% PVA and 1.5 to 10% D-mannitol was applied, the steeply enhanced the adhesion force of the incise drape for measurement was shown as compared with the case where the incise drape for measurement was affixed to an artificial skin to which the test antiseptic solution comprising only 0.5% PVA was applied, or the case where the incise drape for measurement was affixed to an artificial skin to which the test antiseptic solution comprising 0.5% PVA and 1% D-mannitol was applied (see Table 5 and Figure 6).

This result indicates that the present antiseptic composition supplemented with 0.5% of an adhesive agent (PVA), having a sugar alcohol (D-mannitol) concentration adjusted to 1.5 to 10% enhances the adhesion force of an incise drape on skin surface to which the antiseptic composition has been applied, as compared with the case of adjusting the sugar alcohol (D-mannitol) concentration to 1.0% or the case of using a sugar alcohol (D-mannitol)-free antiseptic composition comprising the adhesive agent (PVA). In this study (adhesive agent concentration: 0.5%), any serious problem such as coming off of a film did not occur.

[Table 5]

| Test antiseptic solution | Adhesion force (N/cm) |
|---|---|
| Test antiseptic solution comprising 0.5% PVA | 0.124 ± 0.030 |
| Test antiseptic solution comprising 0.5% PVA and 1% D-mannitol | 0.131 ± 0.028 |
| Test antiseptic solution comprising 0.5% PVA and 1.5% D-mannitol | 0.394 ± 0.060 |
| Test antiseptic solution comprising 0.5% PVA and 2% D-mannitol | 0.398 ± 0.035 |
| Test antiseptic solution comprising 0.5% PVA and 2.5% D-mannitol | 0.393 ± 0.032 |
| Test antiseptic solution comprising 0.5% PVA and 5% D-mannitol | 0.422 ± 0.041 |
| Test antiseptic solution comprising 0.5% PVA and 10% D-mannitol | 0.482 ± 0.029 |

Figure 6 was created on the basis of the results of Table 5.

2-5 Study on concentration of adhesive agent in present antiseptic composition supplemented with adhesive agent - (1)

In order to study whether results similar to those of the above section "2-4" (i.e., the adhesion force enhancing effect based on relationship with the specific concentration of a sugar alcohol) could be obtained by adjusting the concentration of an adhesive agent in the present antiseptic composition supplemented with the adhesive agent to other than 0.5%, the adhesion force of the incise drape for measurement was measured using four types of test antiseptic solutions shown in Table 6.

As a result, by affixing the incise drape for measurement to an artificial skin to which the test antiseptic solution comprising 0.1% PVA and 1.0 to 10.0% D-mannitol was applied, the enhanced the adhesion force was shown. Particularly, when the incise drape for measurement was affixed to an artificial skin to which the test antiseptic solution comprising 0.1% PVA and 1.5 to 10% D-mannitol was applied, the steeply enhanced the adhesion force of the incise drape for measurement was shown as compared with the case where the incise drape for measurement was affixed to an artificial skin to which the test antiseptic solution comprising only 0.1% PVA was applied, or the case where the incise drape for measurement was affixed to an artificial skin to which the test antiseptic solution comprising 0.1% PVA and 1% D-mannitol was applied (see Table 6 and Figure 7).

This result indicates that the present antiseptic composition supplemented with 0.1% of an adhesive agent (PVA), having a sugar alcohol (D-mannitol) concentration adjusted to 1.5 to 10% enhances the adhesion force of an incise drape on skin surface to which the antiseptic composition has been applied, as compared with the case of adjusting the sugar alcohol (D-mannitol) concentration to 1.0% or the case of using a sugar alcohol (D-mannitol)-free antiseptic composition comprising the adhesive agent (PVA), and supports the results of the above section "2-4". In this study (adhesive agent concentration: 0.1%), any serious problem such as coming off of a film did not occur.

[Table 6]

| Test antiseptic solution | Adhesion force (N/cm) |
|---|---|
| Test antiseptic solution comprising 0.1% PVA | 0.047 ± 0.014 |
| Test antiseptic solution comprising 0.1% PVA and 1% D-mannitol | 0.049 ± 0.041 |
| Test antiseptic solution comprising 0.1% PVA and 1.5% D-mannitol | 0.244 ± 0.015 |
| Test antiseptic solution comprising 0.1% PVA and 10% D-mannitol | 0.278 ± 0.046 |

Figure 7 was created on the basis of the results of Table 6.

2-6 Study on concentration of adhesive agent in present antiseptic composition supplemented with adhesive agent - (2)

In order to study whether results similar to those of the above sections "2-4" and "2-5" (i.e., the adhesion force enhancing effect based on relationship with the specific concentration of a sugar alcohol) could be obtained by adjusting the concentration of an adhesive agent in the present antiseptic composition supplemented with the adhesive agent to other than 0.1% and 0.5%, the adhesion force of the incise drape for measurement was measured using four types of test antiseptic solutions shown in Table 7.

As a result, when the incise drape for measurement was affixed to an artificial skin to which the test antiseptic solution comprising 2.0% PVA and 1.5 to 10% D-mannitol was applied, the enhanced the adhesion force of the incise drape for measurement was shown as compared with the case where the incise drape for measurement was affixed to an artificial skin to which the test antiseptic solution comprising 2.0% PVA and 1.0% D-mannitol was applied (see Table 7 and Figure 8).

This result indicates that the present antiseptic composition supplemented with 2.0% of an adhesive agent (PVA), having a sugar alcohol (D-mannitol) concentration adjusted to 1.5 to 10% enhances the adhesion force of an incise drape on skin surface to which the antiseptic composition has been applied, as compared with the case of adjusting the sugar alcohol (D-mannitol) concentration to 1.0%, and supports the results of the above sections "2-4" and "2-5". In this study (adhesive agent concentration: 2.0%), any serious problem such as coming off of a film did not occur.

The results of the above sections "2-4", "2-5", and "2-6" taken together indicate that the present antiseptic composition supplemented with 0.1 to 2.0% of an adhesive agent (PVA), having a sugar alcohol (D-mannitol) concentration adjusted to 1.5 to 10% enhances the adhesion force of an incise drape on skin surface to which the antiseptic composition has been applied, as compared with the case of adjusting the sugar alcohol (D-mannitol) concentration to 1.0% or the case of using a sugar alcohol (D-mannitol)-free antiseptic composition comprising the adhesive agent (PVA).

[Table 7]

| Test antiseptic solution | Adhesion force (N/cm) |
| --- | --- |
| Test antiseptic solution comprising 2% PVA and 1% D-mannitol | $0.232 \pm 0.045$ |
| Test antiseptic solution comprising 2% PVA and 1.5% D-mannitol | $0.324 \pm 0.022$ |
| Test antiseptic solution comprising 2% PVA and 2.5% D-mannitol | $0.593 \pm 0.030$ |
| Test antiseptic solution comprising 2% PVA and 10% D-mannitol | $0.608 \pm 0.025$ |

Figure 8 was created on the basis of the results of Table 7.

2-7 Confirmation that present antiseptic composition comprising high concentration of adhesive agent is unsuitable as antiseptic composition

In order to confirm that the present antiseptic composition comprising a high concentration of an adhesive agent was unsuitable as an antiseptic composition, the adhesion force of the incise drape for measurement was measured using three types of test antiseptic solutions shown in Table 8.

As a result, when the incise drape for measurement was affixed to an artificial skin to which the test antiseptic solution comprising 3.5% PVA and 1.0% or 1.5% D-mannitol was applied, a tendency to reduce the adhesion force of the incise drape for measurement in a concentration-dependent manner of D-mannitol was shown as compared with the case where the incise drape for measurement was affixed to an artificial skin to which the test antiseptic solution comprising only 3.5% PVA was applied (see Table 8 and Figure 9).

This result indicates that unlike the present antiseptic composition comprising a low concentration (e.g., 0.1 to 2.0%) of an adhesive agent, the present antiseptic composition comprising a high concentration (e.g., 3.5%) of the adhesive agent reduces the adhesion force of an incise drape on skin surface to which the antiseptic composition has been applied, due to a sugar alcohol contained therein. The test antiseptic solution comprising 3.5% PVA increased the amount of PVA applied and formed a thick film on the skin surface at the time of drying, though the incise drape had large adhesion force. As a result, the film of the antiseptic composition partially came off. As a matter of course, if an incise drape is affixed to such a film of the antiseptic composition that has come off, no adhesion force to the skin exists. Therefore, particularly, when the film of the antiseptic composition has come off wholly, the overall the adhesion force of an incise drape to the skin might be reduced. The adhesive agent contained at a high concentration in an antiseptic composition elevates the viscosity of the antiseptic composition, and the resulting antiseptic composition probably has poor applicability depending on a purpose. Furthermore, the adhesive agent contained at a high concentration in an antiseptic composition probably has a harmful effect of time-consuming drying after application of the antiseptic composition. Hence, in the case of adding an adhesive agent to the present antiseptic composition, it is probably preferred to adjust the concentration of the adhesive agent so as not to be high.

[Table 8]

| Test antiseptic solution | Adhesion force (N/cm) |
|---|---|
| Test antiseptic solution comprising 3.5% PVA | 0.454 $\pm$ 0.024 |
| Test antiseptic solution comprising 3.5% PVA and 1% D-mannitol | 0.361 $\pm$ 0.015 |
| Test antiseptic solution comprising 3.5% PVA and 1.5% D-mannitol | 0.194 $\pm$ 0.026 |

Figure 9 was created on the basis of the results of Table 8.

2-8 Study on adhesive agent other than PVA to be added to present antiseptic composition

In order to study an adhesive agent other than PVA to be added to the present antiseptic composition, the adhesion force of the incise drape for measurement was measured using five types of test antiseptic solutions shown in Table 9.

As a result, when the incise drape for measurement was affixed to an artificial skin to which the test antiseptic solution comprising 0.5% PVP and 5.0% D-mannitol was applied, the enhanced the adhesion force of the incise drape for measurement was shown as compared with the case where the incise drape for measurement was affixed to an artificial skin to which the test antiseptic solution comprising 0.5% PVP was applied (see Table 9 and Figure 10).

This result indicates that the present antiseptic composition supplemented with an adhesive agent (PVP) other than PVA can also further enhance the adhesion force of an incise drape on skin surface to which the antiseptic composition has been applied, as compared with the present antiseptic composition free of the adhesive agent (PVP).

[Table 9]

| Test antiseptic solution | Adhesion force (N/cm) |
|---|---|
| Test antiseptic solution comprising 5% D-mannitol | 0.217 $\pm$ 0.030 |
| Test antiseptic solution comprising 0.5% PVP | 0.113 $\pm$ 0.028 |
| Test antiseptic solution comprising 0.5% PVA | 0.124 $\pm$ 0.030 |
| Test antiseptic solution comprising 0.5% PVP and 5% D-mannitol | 0.497 $\pm$ 0.067 |
| Test antiseptic solution comprising 0.5% PVA and 5% D-mannitol | 0.447 $\pm$ 0.060 |

Figure 10 was created on the basis of the results of Table 9.

2-9 Study on sugar alcohol other than D-mannitol in present antiseptic composition

In order to study a sugar alcohol other than D-mannitol in the present antiseptic composition, the adhesion force of the incise drape for measurement was measured using three types of test antiseptic solutions shown in Table 10.

As a result, when the incise drape for measurement was affixed to an artificial skin to which the test antiseptic solution comprising 5.0% erythritol was applied, the enhanced the adhesion force of the incise drape for measurement was shown as compared with the case where the incise drape for measurement was affixed to an artificial skin to which the erythritol-free test antiseptic solution (erythritol- and PVA-free test antiseptic solution) was applied (see Table 10 and Figure 11). When the incise drape for measurement was affixed to an artificial skin to which the test antiseptic solution comprising 0.5% PVA and 5.0% erythritol was applied, the further enhanced the adhesion force of the incise drape for measurement was shown as compared with the case where the incise drape for measurement was affixed to an artificial skin to which the test antiseptic solution comprising 5.0% erythritol was applied (see Table 10 and Figure 11).

These results indicate that the present antiseptic composition supplemented with a selected sugar alcohol other than D-mannitol can also enhance the adhesion force of an incise drape on skin surface to which the antiseptic composition has been applied. Both D-mannitol and erythritol are sugar alcohols that have relatively high CRH (specifically, the CRH of D-mannitol is approximately 98, and the CRH of erythritol is approximately 90) and have relatively low moisture absorbency. This suggests a possibility that these sugar alcohols are involved in maintaining the dry state of skin surface after application of the present antiseptic composition, so that the adhesion force of an incise drape is more enhanced.

[Table 10]

| Test antiseptic solution | Adhesion force (N/cm) |
|---|---|
| Erythritol- and PVA-free test antiseptic solution | 0.037 ± 0.007 |
| Test antiseptic solution comprising 5% erythritol | 0.073 ± 0.012 |
| Test antiseptic solution comprising 0.5% PVA and 5% erythritol | 0.165 ± 0.056 |

Figure 11 was created on the basis of the results of Table 10.

2-10 Effect of sugar alcohol in present antiseptic composition under various humidity conditions

In order to confirm that D-mannitol was effective in the present antiseptic composition under humidity conditions other than 50%, the adhesion force of the incise drape for measurement was measured using four types of test antiseptic solutions shown in Table 11 under five types (20%, 30%, 40%, 50%, and 60%) of humidity conditions. In this test, the width of the incise drape was 5 cm; the amount of the test antiseptic solution applied was 270 μL; the application range to an artificial skin was 610 mm × 620 mm; the test antiseptic solution was applied using a spatula, and the incise drape was pressure-bonded and then left standing for 3 minutes; the measurement equipment of the tensile test was Tensilon universal testing machine (model: RTG-1225, manufactured by A&D Co., Ltd.); and the pulling rate was 5.0 mm/sec.

As a result, when the incise drape for measurement was affixed to an artificial skin to which the test antiseptic solution comprising D-mannitol was applied, the enhanced the adhesion force of the incise drape for measurement was shown under all the humidity conditions as compared with the case where the incise drape for measurement was affixed to an artificial skin to which the D-mannitol-free test antiseptic solution was applied, and the suppression of reduction in the adhesion force caused by elevation in humidity was also shown as compared with the case where the incise drape for measurement was affixed to an artificial skin to which the test antiseptic solution comprising PVA was applied (see Table 11 and Figure 12). When the incise drape for measurement was affixed to an artificial skin to which the test antiseptic solution comprising both PVA and D-mannitol was applied, the further suppression of reduction in the adhesion force caused by elevation in humidity was shown as compared with the case where the incise drape for measurement was affixed to an artificial skin to which the test antiseptic solution comprising D-mannitol alone or the test antiseptic solution comprising PVA alone was applied.

These results indicate that a sugar alcohol (D-mannitol) in the present antiseptic composition and the present antiseptic composition supplemented with an adhesive agent (PVA) are also effective under various humidity conditions.

[Table 11]

| Test antiseptic solution | Humidity (%) | Adhesion force (N/cm) |
|---|---|---|
| D-mannitol- and pva-free test antiseptic solution | 20 | 0.167 ± 0.085 |
| | 30 | 0.290 ± 0.015 |
| | 40 | 0.345 ± 0.049 |
| | 50 | 0.037 ± 0.006 |
| | 60 | 0.047 ± 0.003 |
| Test antiseptic solution comprising 0.5% PVA | 20 | 0.611 ± 0.067 |
| | 30 | 0.476 ± 0.153 |
| | 40 | 0.324 ± 0.134 |
| | 50 | 0.100 ± 0.042 |
| | 60 | 0.035 ± 0.013 |

(continued)

| Test antiseptic solution | Humidity (%) | Adhesion force (N/cm) |
|---|---|---|
| Test antiseptic solution comprising 5% D-mannitol | 20 | 0.644 ± 0.152 |
| | 30 | 0.342 ± 0.060 |
| | 40 | 0.533 ± 0.046 |
| | 50 | 0.461 ± 0.058 |
| | 60 | 0.209 ± 0.069 |
| Test antiseptic solution comprising 0.5% PVA and 5% D-mannitol | 20 | 0.621 ± 0.038 |
| | 30 | 0.557 ± 0.011 |
| | 40 | 0.570 ± 0.026 |
| | 50 | 0.421 ± 0.071 |
| | 60 | 0.453 ± 0.084 |

Figure 12 was created on the basis of the results of Table 11.

Example 2. Evaluation of microbicidal activity of present antiseptic composition

In order to examine influence on the microbicidal activity of the present antiseptic composition or the present antiseptic composition supplemented with an adhesive agent, microbicidal activity against bacteria *(Staphylococcus aureus, Staphylococcus epidermidis* and *Pseudomonas aeruginosa)* and a fungus (*Candida albicans*) known to cause infectious disease was evaluated using D-mannitol as a sugar alcohol and using PVA as the adhesive agent.

1. Material and method

1-1 Test antiseptic solution

The test antiseptic solutions used were Olanedine antiseptic solution (2) shown in Table 12 below, supplemented with varying concentrations (1.0%, 2.0%, or 5.0%) of D-mannitol (manufactured by FUJIFILM Wako Pure Chemical Corp.) and/or varying concentrations (1.0% or 2.0%) of PVA (500) (average degree of polymerization: approximately 500, partial saponification product, manufactured by FUJIFILM Wako Pure Chemical Corp.) or varying concentrations (0.5%, 1.0%, or 2.0%) of PVA (1500) (average degree of polymerization: approximately 1500, partial saponification product, manufactured by FUJIFILM Wako Pure Chemical Corp.).

[Table 12]

| Components of Olanedine antiseptic solution (2) and their quantities | |
|---|---|
| Component | Quantity (concentration) |
| Olanexidine gluconate (active ingredient) | 1.5% |
| Polyoxyethylene (20) polyoxypropylene (20) glycol (solubilizing agent) | 1.08% |
| Lauromacrogol (solubilizing agent) | 2.0% |
| Yellow No. 5 (Sunset Yellow) (colorant) | 0.1% |
| Glucono-6-lactone (pH adjuster) | q.s. |
| Sodium hydroxide (pH adjuster) | q.s. |
| Purified water | q.s. |

1-2 Culture medium

1-2-1 SCD plate

1000 mL of pure water was added to 40 g of SCD agar medium "Daigo" (product number: 396-00175, manufactured by Nihon Pharmaceutical Co., Ltd.) and stirred. This mixture was sterilized with high-pressure steam (121°C, 20 min). Before the agar was solidified, approximately 20 mL thereof was dispensed to a petri dish, followed by solidification to prepare SCD plate, which was preserved at room temperature until use.

1-2-2 SCDLP medium

48.0 g of SCDLP agar medium "Daigo" (product code: 398-00255, manufactured by Nihon Pharmaceutical Co., Ltd.) was weighed into a glass Erlenmeyer flask, to which 1000 mL of pure water was then added and stirred. This mixture was sterilized with high-pressure steam (121°C, 20 min) to prepare SCDLP medium, which was preserved in a hot bath set to 47°C until use.

1-2-3 SAB plate

1000 mL of pure water was added to 65 g of Sabouraud agar medium "Nissui" (product number: 05701, manufactured by Nissui Pharmaceutical Co., Ltd.) and stirred. This mixture was sterilized with high-pressure steam (121°C, 20 min). Before the agar was solidified, approximately 20 mL thereof was dispensed to a petri dish, followed by solidification to prepare SAB plate, which was preserved at room temperature until use.

1-2-4 SABLP medium

73.0 g of Sabouraud glucose LP agar medium "Daigo" (product code: 392-01875, manufactured by Nihon Pharmaceutical Co., Ltd.) was weighed into a glass Erlenmeyer flask, to which 1000 mL of pure water was then added and stirred. This mixture was sterilized with high-pressure steam (121°C, 20 min) to prepare SABLP medium, which was preserved in a hot bath set to 47°C until use.

1-3 Reagent

1-3-1 PBS

3.4 g of potassium dihydrogen phosphate was dissolved by the addition of approximately 50 mL of distilled water. The pH of the solution was adjusted to $7.2 \pm 0.2$ by the addition of an aqueous sodium hydroxide solution, and distilled water was then added thereto such that the total amount was 100 mL. The resultant was used as a phosphate buffered preservation solution. 17.5 g of sodium chloride was dissolved by the addition of 2.5 mL of the phosphate buffered preservation solution and approximately 1800 mL of distilled water. Distilled water was added thereto such that the total amount was 2000 mL, followed by high-pressure steam sterilization (121°C, 20 min) to prepare PBS, which was preserved at room temperature until use.

1-3-2 Neutralizing agent

Approximately 800 mL of distilled water was added to 100 g of polysorbate 80 and stirred. 5.0 g of sodium thiosulfate hydrate, 0.4 g of potassium dihydrogen phosphate, 1 mL of Triton X-100, 10.1 g of anhydrous sodium monohydrogen phosphate, and 11.7 g of soybean lecithin were added thereto and stirred. Further, 10.0 g of Tamol(R) NN8906 was added thereto, and heated and stirred until dissolved. The solution was cooled to room temperature, and its pH was then adjusted to 7.8 to 7.9 by the addition of a 1 mol/L sodium hydroxide solution. Distilled water was added thereto such that the total amount was 1000 mL. Then, the mixture was transferred to a medium bottle and sterilized with high-pressure steam. The mixture thus sterilized was taken out of the high-pressure steam sterilizer and cooled to room temperature with stirring to prepare a neutralizing agent.

1-4 Preculture of test microbe and preparation of test microbial suspension

The test microbes were *Staphylococcus aureus* ATCC29213, *Staphylococcus epidermidis* ATCC12228, *Pseudomonas aeruginosa* ATCC27853, and a fungus *Candida albicans* ATCC90028. Each test microbe was cultured in the SCD plate (*Staphylococcus aureus, Staphylococcus epidermidis* and *Pseudomonas aeruginosa*) or the SAB plate (*Can-*

*dida albicans*) and then suspended in distilled water, and a 5-fold dilution of the microbial suspension with McFarland 1 (*Staphylococcus aureus, Staphylococcus epidermidis* and *Pseudomonas aeruginosa)* or a test microbial suspension with McFarland 5 (*Candida albicans*) was prepared.

1-5 Microbicidal activity evaluation test

1-5-1 Measurement of the number of viable microbes (*Staphylococcus aureus, Staphylococcus epidermidis,* and *Pseudomonas aeruginosa)* before action of test antiseptic solution

The number of viable microbes (*Staphylococcus aureus, Staphylococcus epidermidis,* and *Pseudomonas aeruginosa)* before action of each test antiseptic solution was measured according to the following procedures [1] to [6].

[1] 150 $\mu$L of the test microbial suspension of *Staphylococcus aureus, Staphylococcus epidermidis,* or *Pseudomonas aeruginosa* was added to 3 mL of PBS and mixed.
[2] Immediately thereafter, 500 $\mu$L of the microbial mixture was added to 4.5 mL of a neutralizing agent and mixed. This mixture was used as a $10^1$-fold dilution.
[3] The $10^1$-fold dilution was diluted 10-fold with a neutralizing agent. Dilution was further repeated to produce a 10-fold dilution series (a total 5 stages from $10^1$- to $10^5$-fold dilutions) .
[4] 1 mL each of $10^3$- to $10^5$-fold dilutions was dispensed to a petri dish, and approximately 20 mL of the SCDLP medium preserved in a hot bath was added thereto to produce a pour plate.
[5] The pour plate was solidified, then inverted, and aerobically cultured at $35 \pm 2$°C for 2 days.
[6] Colonies that proliferated in the pour plate were visually counted, and the number of colonies was multiplied by the dilution ratio to calculate the number of viable microbes (CFU/mL) (n = 3).

1-5-2 Measurement of the number of viable microbes (*Staphylococcus aureus, Staphylococcus epidermidis,* and *Pseudomonas aeruginosa)* after action of test antiseptic solution

The number of viable microbes (*Staphylococcus aureus, Staphylococcus epidermidis,* and *Pseudomonas aeruginosa)* after action of each test antiseptic solution was measured according to the following procedures [1] to [5].

[1] 150 $\mu$L of the test microbial suspension of *Staphylococcus aureus, Staphylococcus epidermidis,* or *Pseudomonas aeruginosa* was added to 3 mL each of ten types of test antiseptic solutions (a test antiseptic solution comprising 2.0% PVA (500); a test antiseptic solution comprising 1.0% PVA (500); a test antiseptic solution comprising 2.0% PVA (1500); a test antiseptic solution comprising 1.0% PVA (1500); a test antiseptic solution comprising 0.5% PVA (1500); a test antiseptic solution comprising 5.0% D-mannitol; a test antiseptic solution comprising 2.0% D-mannitol; a test antiseptic solution comprising 1.0% D-mannitol; a test antiseptic solution comprising 2.0% PVA (500) and 5.0% D-mannitol; and a test antiseptic solution comprising 1.0% PVA (1500) and 5.0% D-mannitol) and mixed. This mixture was used as a reaction mixture, and reaction was performed at room temperature.
[2] After reaction for a predetermined time (30 sec or 60 sec), 500 $\mu$L of each reaction mixture was extracted, added to 4.5 mL of a neutralizing agent, and mixed. This mixture was used as a $10^1$-fold dilution.
[3] 1 mL of each $10^1$-fold dilution was dispensed to a petri dish, and approximately 20 mL of the SCDLP medium preserved in a hot bath was added thereto to produce a pour plate.
[4] The pour plate was solidified, then inverted, and aerobically cultured at $35 \pm 2$°C for 2 days.
[5] Colonies that proliferated in the pour plate were visually counted, and the number of colonies was multiplied by the dilution ratio to calculate the number of viable microbes (CFU/mL) (n = 3).

1-5-3 Measurement of the number of viable microbes (*Candida albicans*) before action of test antiseptic solution

The number of viable microbes (*Candida albicans*) before action of each test antiseptic solution was measured according to the following procedures [1] to [6].

[1] 150 $\mu$L of the test microbial suspension of *Candida albicans* was added to 3 mL of PBS and mixed.
[2] Immediately thereafter, 500 $\mu$L of the microbial mixture was added to 4.5 mL of a neutralizing agent and mixed. This mixture was used as a $10^1$-fold dilution.
[3] The $10^1$-fold dilution was diluted 10-fold with a neutralizing agent. Dilution was further repeated to produce a 10-fold dilution series (a total 4 stages from $10^1$- to $10^4$-fold dilutions) .
[4] 1 mL each of $10^2$- to $10^4$-fold dilutions was dispensed to a petri dish, and approximately 20 mL of the SABLP medium preserved in a hot bath was added thereto to produce a pour plate.

[5] The pour plate was solidified, then inverted, and aerobically cultured at 35 ± 2°C for 2 days.
[6] Colonies that proliferated in the pour plate were visually counted, and the number of colonies was multiplied by the dilution ratio to calculate the number of viable microbes (CFU/mL) (n = 3).

1-5-4 Measurement of the number of viable microbes (*Candida albicans*) after action of test antiseptic solution

The number of viable microbes (*Candida albicans*) after action of each test antiseptic solution was measured according to the following procedures [1] to [6].

[1] 150 μL of the test microbial suspension of *Candida albicans* was added to 3 mL each of the ten types of test antiseptic solutions described above, and mixed. This mixture was used as a reaction mixture, and reaction was performed at room temperature.
[2] After reaction for a predetermined time (30 sec or 60 sec), 500 μL of each reaction mixture was extracted, added to 4.5 mL of a neutralizing agent, and mixed. This mixture was used as a $10^1$-fold dilution.
[3] The $10^1$-fold dilution was diluted 10-fold with a neutralizing agent. Dilution was further repeated to produce a 10-fold dilution series (a total 3 stages from $10^1$- to $10^3$-fold dilutions) .
[4] 1 mL of each $10^2$- or $10^3$-fold dilution was dispensed to a petri dish, and approximately 20 mL of the SABLP medium preserved in a hot bath was added thereto to produce a pour plate.
[5] The pour plate was solidified, then inverted, and aerobically cultured at 35 ± 2°C for 2 days.
[6] Colonies that proliferated in the pour plate were visually counted, and the number of colonies was multiplied by the dilution ratio to calculate the number of viable microbes (CFU/mL) (n = 3).

1-5-5 Calculation of microbicidal activity

If no colony was detected after action of the test antiseptic solution, the lower detection limit was 10 CFU/mL for *Staphylococcus aureus, Staphylococcus epidermidis* and *Pseudomonas aeruginosa* and 100 CFU/mL for *Candida albicans.* Common logarithmic values of the number of viable microbes (CFU/mL) before action of the test antiseptic solution and the number of viable microbes after action of the test antiseptic solution for each reaction time (30 sec or 60 sec) were determined. Microbicidal activity was indicated by a relative value with microbicidal activity without detectable colonies defined as 1 (see the following equation).

$$\text{Microbicidal activity} = (A - B) / (A - C)$$

A: Average number of viable microbes (common logarithmic value) before action of the test antiseptic solution
B: The number of viable microbes (common logarithmic value) after action of the test antiseptic solution
C: Common logarithmic value of the lower limit value of detection (the number of viable microbes when no colony was detected)

2. Results

When three strains of bacteria (*Staphylococcus aureus, Staphylococcus epidermidis,* and *Pseudomonas aeruginosa*) were each reacted for 30 seconds or 60 seconds using three types of present compositions for antisepsis (a test antiseptic solution comprising 1.0% D-mannitol, a test antiseptic solution comprising 2.0% D-mannitol, and a test antiseptic solution comprising 5.0% D-mannitol) and two types of present compositions for antisepsis supplemented with an adhesive agent (a test antiseptic solution comprising 2.0% PVA (500) and 5.0% D-mannitol, and a test antiseptic solution comprising 1.0% PVA (1500) and 5.0% D-mannitol), no surviving microbe was detected for any of the bacteria (see Tables 13 to 15). When a fungus (*Candida albicans*) was reacted for 30 seconds or 60 seconds using the two types of present compositions for antisepsis supplemented with an adhesive agent, a tendency of strong microbicidal activity was shown as compared with the case of using a test antiseptic solution comprising only PVA (500) or PVA (1500) or the case of using the three types of present compositions for antisepsis (see Table 16).
These results indicate that a sugar alcohol in the present antiseptic composition or an adhesive agent to be added to the present antiseptic composition does not adversely affect the microbicidal activity of the antiseptic composition, and also indicates that the addition of an adhesive agent to the present antiseptic composition tends to increase microbicidal activity as compared with the case of adding no adhesive agent. Particularly, *Candida albicans* is known as a high-risk microbe for hospital-acquired infection. The observed enhancing effect on microbicidal activity against *Candida albicans* is considered to be of very great clinical significance.

[Table 13]

| Microbicidal activity against Staphylococcus aureus ATCC29213 | | |
|---|---|---|
| Test antiseptic solution | Microbicidal activity | |
| | 30 sec | 60 sec |
| Test antiseptic solution comprising 2% PVA (500) | 1.00 ± 0.00 | 1.00 ± 0.00 |
| Test antiseptic solution comprising 1% PVA (500) | 1.00 ± 0.00 | 1.00 ± 0.00 |
| Test antiseptic solution comprising 2% PVA (1500) | 1.00 ± 0.00 | 1.00 ± 0.00 |
| Test antiseptic solution comprising 1% PVA (1500) | 1.00 ± 0.00 | 1.00 ± 0.00 |
| Test antiseptic solution comprising 0.5% PVA (1500) | 1.00 ± 0.00 | 1.00 ± 0.00 |
| Test antiseptic solution comprising 5.0% D-mannitol | 1.00 ± 0.00 | 1.00 ± 0.00 |
| Test antiseptic solution comprising 2.0% D-mannitol | 1.00 ± 0.00 | 1.00 ± 0.00 |
| Test antiseptic solution comprising 1.0% D-mannitol | 1.00 ± 0.00 | 1.00 ± 0.00 |
| Test antiseptic solution comprising 2% PVA (500) and 5% D-mannitol | 1.00 ± 0.00 | 1.00 ± 0.00 |
| Test antiseptic solution comprising 1% PVA (1500) and 5% D-mannitol | 1.00 ± 0.00 | 1.00 ± 0.00 |

[Table 14]

| Microbicidal activity against Staphylococcus epidermidis ATCC12228 | | |
|---|---|---|
| Test antiseptic solution | Microbicidal activity | |
| | 30 sec | 60 sec |
| Test antiseptic solution comprising 2% PVA (500) | 1.00 ± 0.00 | 1.00 ± 0.00 |
| Test antiseptic solution comprising 1% PVA (500) | 1.00 ± 0.00 | 1.00 ± 0.00 |
| Test antiseptic solution comprising 2% PVA (1500) | 1.00 ± 0.00 | 1.00 ± 0.00 |
| Test antiseptic solution comprising 1% PVA (1500) | 1.00 ± 0.00 | 1.00 ± 0.00 |
| Test antiseptic solution comprising 0.5% PVA (1500) | 1.00 ± 0.00 | 1.00 ± 0.00 |
| Test antiseptic solution comprising 5.0% D-mannitol | 1.00 ± 0.00 | 1.00 ± 0.00 |
| Test antiseptic solution comprising 2.0% D-mannitol | 1.00 ± 0.00 | 1.00 ± 0.00 |
| Test antiseptic solution comprising 1.0% D-mannitol | 1.00 ± 0.00 | 1.00 ± 0.00 |
| Test antiseptic solution comprising 2% PVA (500) and 5% D-mannitol | 1.00 ± 0.00 | 1.00 ± 0.00 |
| Test antiseptic solution comprising 1% PVA (1500) and 5% D-mannitol | 1.00 ± 0.00 | 1.00 ± 0.00 |

[Table 15]

| Microbicidal activity against Pseudomonas aeruginosa ATCC27853 | | |
|---|---|---|
| Test antiseptic solution | Microbicidal activity | |
| | 30 sec | 60 sec |
| Test antiseptic solution comprising 2% PVA (500) | 1.00 ± 0.00 | 1.00 ± 0.00 |
| Test antiseptic solution comprising 1% PVA (500) | 1.00 ± 0.00 | 1.00 ± 0.00 |
| Test antiseptic solution comprising 2% PVA (1500) | 1.00 ± 0.00 | 1.00 ± 0.00 |
| Test antiseptic solution comprising 1% PVA (1500) | 1.00 ± 0.00 | 1.00 ± 0.00 |
| Test antiseptic solution comprising 0.5% PVA (1500) | 1.00 ± 0.00 | 1.00 ± 0.00 |

(continued)

| Microbicidal activity against *Pseudomonas aeruginosa* ATCC27853 | | |
|---|---|---|
| Test antiseptic solution | Microbicidal activity | |
| | 30 sec | 60 sec |
| Test antiseptic solution comprising 5.0% D-mannitol | 1.00 ± 0.00 | 1.00 ± 0.00 |
| Test antiseptic solution comprising 2.0% D-mannitol | 1.00 ± 0.00 | 1.00 ± 0.00 |
| Test antiseptic solution comprising 1.0% D-mannitol | 1.00 ± 0.00 | 1.00 ± 0.00 |
| Test antiseptic solution comprising 2% PVA (500) and 5% D-mannitol | 1.00 ± 0.00 | 1.00 ± 0.00 |
| Test antiseptic solution comprising 1% PVA (1500) and 5% D-mannitol | 1.00 ± 0.00 | 1.00 ± 0.00 |

[Table 16]

| Microbicidal activity against *Candida albicans* ATCC90028 | | |
|---|---|---|
| Test antiseptic solution | Microbicidal activity | |
| | 30 sec | 60 sec |
| Test antiseptic solution comprising 2% PVA (500) | 0.18 ± 0.07 | 0.41 ± 0.19 |
| Test antiseptic solution comprising 1% PVA (500) | 0.17 ± 0.06 | 0.38 ± 0.17 |
| Test antiseptic solution comprising 2% PVA (1500) | 0.17 ± 0.06 | 0.35 ± 0.15 |
| Test antiseptic solution comprising 1% PVA (1500) | 0.15 ± 0.07 | 0.32 ± 0.14 |
| Test antiseptic solution comprising 0.5% PVA (1500) | 0.15 ± 0.07 | 0.30 ± 0.14 |
| Test antiseptic solution comprising 5.0% D-mannitol | 0.17 ± 0.06 | 0.33 ± 0.14 |
| Test antiseptic solution comprising 2.0% D-mannitol | 0.15 ± 0.06 | 0.31 ± 0.13 |
| Test antiseptic solution comprising 1.0% D-mannitol | 0.15 ± 0.05 | 0.30 ± 0.11 |
| Test antiseptic solution comprising 2% PVA (500) and 5% D-mannitol | 0.25 ± 0.06 | 0.59 ± 0.23 |
| Test antiseptic solution comprising 1% PVA (1500) and 5% D-mannitol | 0.18 ± 0.06 | 0.43 ± 0.15 |

Example 3. Measurement of adhesion force of incise drape on skin surface to which present antiseptic composition was applied (2)

In Example 1, "3M Ioban Special Incise Drape" was used as incise drape for measurement. In order to confirm whether the adhesion force enhancing effect by the present antiseptic composition is exerted without relying on the types of incise drape, "Opsite Incise" (manufactured by Smith & Nephew) which is an incise drape other than "3M Ioban Special Incise Drape" was used, and analyzed according to the method described in Example 1. Further, since both "3M Ioban Special Incise Drape" and "Opsite Incise" are acrylic adhesive agents, in order to confirm whether the adhesion force enhancing effect by the present antiseptic composition is also exerted when using medical tape other than acrylic adhesive agents, "3M micropore S Kind Removal Silicone Tape (corresponding to "3M™ Micropore™ S Surgical Tape" in the US)" (manufactured by 3M) which is a surgical tape being a silicone-based adhesive agent was similarly analyzed. For "3M micropore S Kind Removal Silicone Tape", it was measured with a width of 2.5 cm, and not a width of 4 cm.

As a result of measuring the adhesion force of "Opsite Incise" using four types of test antiseptic solutions shown in Table 17, it was shown that the present antiseptic solution (i.e. antiseptic composition comprising sugar alcohol "D-mannitol") has a higher adhesion force of "Opsite Incise" on skin surface to which the antiseptic composition has been applied, as compared with the case of using a sugar alcohol (D-mannitol)-free antiseptic composition, and the adhesion force was enhanced to similar level as compared with the case where an antiseptic solution comprising an adhesive agent (PVA) was used (see Table 17 and Figure 13).

Further, it was shown that when "Opsite Incise" was affixed to artificial skin to which test antiseptic solution comprising both PVA and D-mannitol was applied, the adhesion force of "Opsite Incise" was enhanced as compared with the case where "Opsite Incise" was affixed to an artificial skin to which a test antiseptic solution comprising PVA or D-mannitol alone was applied (see Table 17 and Figure 13).

[Table 17]

| Test antiseptic solution | Adhesion force (N/cm) |
|---|---|
| D-mannitol- and pva-free test antiseptic solution | 0.195±0.012 |
| Test antiseptic solution comprising 0.5% PVA | 0.333±0.026 |
| Test antiseptic solution comprising 5% D-mannitol | 0.330±0.027 |
| Test antiseptic solution comprising 0.5% PVA and 5% D-mannitol | 0.457±0.062 |

Figure 13 was created on the basis of the results of Table 17.

Further, as a result of measuring the adhesion force of "Opsite Incise" using four types of test antiseptic solutions shown in Table 18, it was shown that when "Opsite Incise" was affixed to an artificial skin to which the test antiseptic solution comprising 1.0 to 5% D-mannitol was applied, the adhesion force of "Opsite Incise" was enhanced as compared with the case where "Opsite Incise" was affixed to an artificial skin to which a D-mannitol-free test antiseptic solution was applied (see Table 18 and Figure 14) .

[Table 18]

| Test antiseptic solution | Adhesion force (N/cm) |
|---|---|
| D-mannitol-free test antiseptic solution | 0.195±0.012 |
| Test antiseptic solution comprising 1% D-mannitol | 0.298±0.013 |
| Test antiseptic solution comprising 1.5% D-mannitol | 0.282±0.034 |
| Test antiseptic solution comprising 5% D-mannitol | 0.330±0.027 |

Figure 14 was created on the basis of the results of Table 18.

As a result of measuring the adhesion force of "3M micropore S Kind Removal Silicone Tape" using four types of test antiseptic solutions shown in Table 19, it was shown that the present antiseptic solution (i.e. antiseptic composition comprising sugar alcohol "D-mannitol") has a higher adhesion force of "3M S micropore Kind Removal Silicone Tape" on skin surface to which the antiseptic composition has been applied, as compared with the case of using an antiseptic solution each comprising sugar alcohol (D-mannitol) or adhesive agent (PVA) alone (see Table 19 and Figure 15).

Further, by affixing "3M S micropore Kind Removal Silicone Tape" to an artificial skin to which the test antiseptic solution comprising both PVA and D-mannitol was applied, it was shown that the adhesion force of "3M S micropore Kind Removal Silicone Tape" was enhanced, as compared with the case of affixing "3M S micropore Kind Removal Silicone Tape" to an artificial skin to which a test antiseptic solution comprising PVA or D-mannitol alone was applied (see Table 19 and Figure 15).

[Table 19]

| Test antiseptic solution | Adhesion force (N/cm) |
|---|---|
| D-mannitol- and pva-free test antiseptic solution | 0.565±0.046 |
| Test antiseptic solution comprising 0.5% PVA | 0.684±0.019 |
| Test antiseptic solution comprising 5% D-mannitol | 0.902±0.069 |
| Test antiseptic solution comprising 0.5% PVA and 5% D-mannitol | 1.037±0.046 |

Figure 15 was created on the basis of the results of Table 19.

As a result of measuring the adhesion force of "3M S micropore Kind Removal Silicone Tape" using four types of test antiseptic solutions shown in Table 20, it was shown that the adhesion force of "3M S micropore Kind Removal Silicone Tape" was enhanced when "3M S micropore Kind Removal Silicone Tape" was affixed to an artificial skin to which a test antiseptic solution comprising 1.0-5% D-mannitol was applied as compared with the case where "3M S micropore Kind Removal Silicone Tape" was affixed to an artificial skin to which a D-mannitol-free antiseptic solution was applied (see Table 20 and Figure 16).

[Table 20]

| Test antiseptic solution | Adhesion force (N/cm) |
|---|---|
| D-mannitol-free Test antiseptic solution | 0.565±0.046 |
| Test antiseptic solution comprising 1% D-mannitol | 0.667±0.074 |
| Test antiseptic solution comprising 1.5% D-mannitol | 0.840±0.061 |
| Test antiseptic solution comprising 5% D-mannitol | 0.902±0.069 |

Figure 16 was created on the basis of the results of Table 20.

Further, as a result of measuring the adhesion force of "3M S micropore Kind Removal Silicone Tape" using five types of test antiseptic solutions shown in Table 21, enhancement of the adhesion force was shown by affixing "3M S micropore Kind Removal Silicone Tape" to an artificial skin to which a test antiseptic solution comprising 0.5% PVA and 1.5-10.0% D-mannitol was applied (see Table 21 and Figure 17).

[Table 21]

| Test antiseptic solution | Adhesion force (N/cm) |
|---|---|
| Test antiseptic solution comprising 0.5% PVA | 0.684±0.019 |
| Test antiseptic solution comprising 0.5% PVA and 1% D-mannitol | 0.674±0.048 |
| Test antiseptic solution comprising 0.5% PVA and 1.5% D-mannitol | 0.908±0.019 |
| Test antiseptic solution comprising 0.5% PVA and 5% D-mannitol | 1.037±0.046 |
| Test antiseptic solution comprising 0.5% PVA and 10% D-mannitol | 1.019±0.031 |

**Figure** 17 was created on the basis of the results of Table 21.

The above results show that the adhesion force enhancing effect of incise drape by the present antiseptic composition is exerted not relying on the types of incise drape.

**Industrial Applicability**

The present invention contributes to the prevention of infectious disease (e.g., infectious disease ascribable to bacteria, fungi, viruses, or the like) at a wound site or an operation site in surgery.

**Claims**

1. An antiseptic composition comprising a microbicide and a sugar alcohol.

2. The antiseptic composition according to claim 1, wherein the sugar alcohol has an action of enhancing an adhesion force of an incise drape on skin surface after application of the antiseptic composition.

3. The antiseptic composition according to claim 1 or 2, wherein the sugar alcohol is D-mannitol or erythritol.

4. The antiseptic composition according to any one of claims 1 to 3, further comprising an adhesive agent.

5. The antiseptic composition according to claim 4, wherein the adhesive agent has an action of enhancing an adhesion force of an incise drape on skin surface after application of the antiseptic composition.

6. The antiseptic composition according to claim 4 or 5, wherein a concentration of the adhesive agent is 2% (w/v) or less.

7. The antiseptic composition according to any one of claims 4 to 6, wherein the adhesive agent is polyvinyl alcohol or polyvinylpyrrolidone.

8. The antiseptic composition according to any one of claims 1 to 7, wherein the microbicide is olanexidine gluconate.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig.13]

[Fig.14]

[Fig.15]

[Fig.16]

[Fig. 17]

# INTERNATIONAL SEARCH REPORT

| | International application No. |
|---|---|
| | **PCT/JP2022/029618** |

## A. CLASSIFICATION OF SUBJECT MATTER

*A61K 45/00*(2006.01)i; *A61K 31/155*(2006.01)i; *A61K 47/26*(2006.01)i; *A61K 47/32*(2006.01)i; *A61L 2/18*(2006.01)i; *A61P 17/00*(2006.01)i

FI:     A61K45/00; A61P17/00 101; A61K47/32; A61L2/18; A61K47/26; A61K31/155

According to International Patent Classification (IPC) or to both national classification and IPC

## B. FIELDS SEARCHED

Minimum documentation searched (classification system followed by classification symbols)

A61K45/00; A61K31/155; A61K47/26; A61K47/32; A61L2/18; A61P17/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

## C. DOCUMENTS CONSIDERED TO BE RELEVANT

| Category* | Citation of document, with indication, where appropriate, of the relevant passages | Relevant to claim No. |
|---|---|---|
| X | JP 7-506377 A (ALCON LABORATORIES, INC.) 13 July 1995 (1995-07-13)<br>claims 1-11, examples 1-12 | 1-7 |
| X | CN 106668850 A (HORIZON INTERNATIONAL (BEIJING) MEDICAL DEVICES CO., LTD.) 17 May 2017 (2017-05-17)<br>claims 1-3, compositions 4, 6 | 1-7 |
| X | JP 2009-242348 A (KOBAYASHI PHARMACEUTICAL CO., LTD.) 22 October 2009 (2009-10-22)<br>claim 1, paragraph [0016], examples 1, 3, 5, 7-40 | 1–7 |
| X | オラネジン消毒液1.5%,同液1.5%消毒用アプリケータ10mL,同液1.5%消毒用アプリケータ25mL. 審議結果報告書. [online], 2015, pp. 20, 21, [retrieved on 28 September 2022], Internet: <URL: https://www.pmda.go.jp/drugs/2015/P20150716001/180079000_22700AMX00707_A100_1.pdf>, non-official translation (Olanedine Antiseptic Solution 1.5%, Olanedine Solution 1.5% Antiseptic Applicator 10mL, Olanedine Solution 1.5% Antiseptic Applicator 25 mL. Report of Review Outcome.)<br>p. 20, line 33 to p. 21, line 4 | 1-8 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 September 2022** | **11 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
|---|
| **PCT/JP2022/029618** |

**C.    DOCUMENTS CONSIDERED TO BE RELEVANT**

| Category* | Citation of document, with indication, where appropriate, of the relevant passages | Relevant to claim No. |
|---|---|---|
| A | CN 107668062 A (JINAN BOSHI MEDICINE TECHNOLOGY CO., LTD.) 09 February 2018 (2018-02-09)<br>    claims 1-13, paragraph [0087] | 1-8 |

Form PCT/ISA/210 (second sheet) (January 2015)

INTERNATIONAL SEARCH REPORT
Information on patent family members

| International application No. |
| --- |
| **PCT/JP2022/029618** |

| Patent document cited in search report | | | Publication date (day/month/year) | Patent family member(s) | Publication date (day/month/year) |
| --- | --- | --- | --- | --- | --- |
| JP | 7-506377 | A | 13 July 1995 | WO 1993/021903 A1 claims 1-11, examples 1-12 EP 639070 A1 US 5342620 A | |
| CN | 106668850 | A | 17 May 2017 | (Family: none) | |
| JP | 2009-242348 | A | 22 October 2009 | (Family: none) | |
| CN | 107668062 | A | 09 February 2018 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6049423 A

- JP 2000159633 A

**Non-patent literature cited in the description**

- *Practical Guidelines for Operative Medicine (revised)*

- Practical Guidelines for Operative Medicine. *Journal of Japanese Association for Operative Medicine,* 2013, vol. 35